(19) Europäisches Patentamt
European Patent Office
Office européen des brevets

(11) Veröffentlichungsnummer: **0 428 665 B1**

# (12) EUROPÄISCHE PATENTSCHRIFT

(45) Veröffentlichungstag der Patentschrift: **05.04.95**

(51) Int. Cl.[6]: **C09K 19/34**, C07D 239/34

(21) Anmeldenummer: **90908217.4**

(22) Anmeldetag: **25.05.90**

(86) Internationale Anmeldenummer:
**PCT/EP90/00846**

(87) Internationale Veröffentlichungsnummer:
**WO 90/15116 (13.12.90 90/28)**

Die Akte enthält technische Angaben, die nach dem Eingang der Anmeldung eingereicht wurden und die nicht in dieser Patentschrift enthalten sind.

## (54) FLUORPHENYLPYRIMIDINE.

(30) Priorität: **10.06.89 DE 3919104**

(43) Veröffentlichungstag der Anmeldung:
**29.05.91 Patentblatt 91/22**

(45) Bekanntmachung des Hinweises auf die Patenterteilung:
**05.04.95 Patentblatt 95/14**

(84) Benannte Vertragsstaaten:
**DE GB NL**

(56) Entgegenhaltungen:
**EP-A- 0 126 949**
**EP-A- 0 260 077**
**WO-A-86/07055**

(73) Patentinhaber: **MERCK PATENT GmbH**
**Postfach,**
**Frankfurter Strasse 250**
**D-64271 Darmstadt (DE)**

(72) Erfinder: **WÄCHTLER, Andreas**
**Goethestrasse 34**
**D-6103 Griesheim (DE)**
Erfinder: **KOMPTER, Hans-Michael**
**Mainzer Landstrasse 14**
**D-6108 Weiterstadt (DE)**
Erfinder: **POETSCH, Eike**
**Am Buchwald 4**
**D-6109 Mühltal 6 (DE)**
Erfinder: **GEELHAAR, Thomas**
**Trajanstrasse 12**
**D-6500 Mainz (DE)**
Erfinder: **HITTICH, Reinhard**
**Am Kirchberg 11**
**D-6101 Modautal 1 (DE)**
Erfinder: **KRAUSE, Joachim**
**Samuel-Morse-Strasse 14**
**D-6110 Dieburg (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 428 665 B1

**Beschreibung**

Die Erfindung betrifft Fluorphenylpyrimidine der Formel I

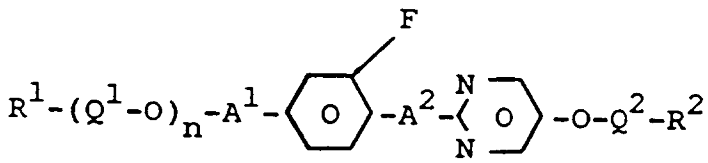

I

wobei

| | |
|---|---|
| $Q^1$ und $Q^2$ | jeweils unabhängig voneinander CO oder $CH_2$, |
| n | 0 oder 1, |
| $A^1$ und $A^2$ | jeweils unabhängig Voneinander 1,4-Phenylen oder eine Einfachbindung, und |
| $R^1$ und $R^2$ | jeweils unabhängig Voneinander ein unsubstituiertes oder mit CN oder mit mindestens einem Halogen substituierter Alkyl- oder Alkenylrest mit bis zu 18 C-Atomen, worin eine oder mehrere $CH_2$-Gruppen ersetzt sein können durch einen Rest ausgewählt aus der Gruppe -O-, -CO-O-, -O-CO- oder -C≡C- wobei zwei Sauerstoffatome nicht benachbart sind, |

bedeuten, und im Falle n = 1
einer der Reste $R^1$ und $R^2$ auch eine Gruppe der Formel II bedeuten kann,

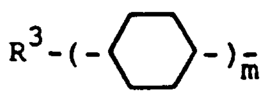

II

worin

| | |
|---|---|
| $R^3$ | eine unsubstituierte oder mit CN oder mit mindestens einem Halogen substituierte Alkyl-, Alkenyl- oder Alkoxygruppe mit bis zu 18 C-Atomen bedeutet, und |
| m | 1 oder 2 ist. |

Chirale getiltete smektische flüssigkristalline Phasen mit ferroelektrischen Eigenschaften können hergestellt werden, indem man Basis-Mischungen mit einer oder mehreren getilteten smektischen Phasen mit einem geeigneten chiralen Dotierstoff versetzt (L.A. Beresnev et al., Mol. Cryst. Liq. Cryst. 89, 327 (1982); H.R. Brand et al., J. Physique 44 (lett.), L 771 (1983). Solche Phasen können als Dielektrika für schnell schaltende Displays verwendet werden, die auf dem von Clark und Lagerwall beschriebenen Prinzip der SSFLC-Technologie (N.A. Clark und S.T. Lagerwall, Appl. Phys. Lett. 36, 899 (1980); USP 4,367.924) auf der Basis der ferroelektrischen Eigenschaften der chiral getilteten Phase beruhen. In dieser Phase sind die langgestreckten Moleküle in Schichten angeordnet, wobei die Moleküle einen Tiltwinkel zur Schichtennormalen aufweisen. Beim Fortschreiten von Schicht zu Schicht ändert sich die Tiltrichtung um einen kleinen Winkel bezüglich einem senkrecht zu den Schichten steherden Achse, so daß eine Helixstruktur ausgebildet wird. In Displays, die auf dem Prinzip der SSFLC-Technologie beruhen, sind die smektischen Schichten senkrecht zu den Platten der Zelle angeordnet. Die helixartige Anordnung der Tiltrichtungen der Moleküle wird durch einen sehr geringen Abstand der Platten (ca. 1-2 μm) unterdrückt. Dadurch werden die Längsachsen der Moleküle gezwungen, sich in einer Ebene parallel zu den Platten der Zelle anzuordnen, wodurch zwei ausgezeichnete Tiltorientierungen entstehen. Durch Anlegen eines geeigneten elektrischen Wechselfeldes kann in der eine spontane Polarisation aufweisenden flüssigkristallinen Phase zwischen diesen beiden Zuständen hin- und hergeschaltet werden. Dieser Schaltvorgang ist wesentlich schneller als bei herkömmlichen verdrillten Zellen (TN-LCD's), die auf nematischen Flüssigkristallen basieren.

Ein großer Nachteil für viele Anwendungen der derzeit verfügbaren Materialien mit chiral getilteten smektischen Phasen (wie z.B. $S_C^*$, jedoch auch $S_H^*$, $S_I^*$, $S_J^*$, $S_K^*$, $S_G^*$, $S_F^*$) ist deren geringe chemische, thermische und Photo-Stabilität. Eine weitere nachteilige Eigenschaft von Displays basierend auf derzeit verfügbaren chiral getilteten smektischen Mischungen ist, daß die Spontanpolarisation zu kleine Werte aufweist, so daß das Schaltzeitverhalten der Displays ungünstig beeinflußt wird und/oder der Pitch und/oder der Tilt und/oder die Viskosität der Phasen nicht den Anforderungen der Display-Technologie entspricht. Darüberhinaus ist meist der Temperaturbereich der ferroelektrischen Phasen zu klein und liegt überwiegend

bei zu hohen Temperaturen.

Es wurde nun gefunden, daß die Verwendung von Verbindungen der Formel I als Komponenten chiral getilteter smektischer Mischungen die erwähnten Nachteile wesentlich vermindern kann. Die Verbindungen der Formel I sind somit als Komponenten chiral getilteter smektischer flüssigkristalliner Phasen vorzüglich geeignet. Insbesondere sind mit ihrer Hilfe chemisch besonders stabile chiral getiltete smektische flüssigkristalline Phasen mit günstigen ferroelektrischen Phasenbereichen, günstigen Weiten für die Viskosität, insbesondere mit breiten $S_C{}^*$ Phasenbereichen, hervorragender Unterkühlbarkeit bis zu Temperaturen unter 0 °C ohne daß Kristallisation auftritt und für derartige Phasen hohen Werten für die spontane Polarisation herstellbar. P ist die spontane Polarisation in $nC/cm^2$. Die Verbindungen der Formel I eignen sich jedoch auch für flüssigkristalline Phasen für den elektroklinen Effekt.

Die Verbindungen der Formel I weisen eine neutrale Anisotropie der Dielektrizitätskonstanten auf ($\Delta\epsilon$ = -0,2 bis +0,5),besitzen daher einen breiten Anwendungsbereich. In Abhängigkeit von der Auswahl der Substituenten können diese Verbindungen als Basismaterialien dienen, aus denen flüssigkristalline smektische Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie und/oder die Viskosität und/oder die spontane Polarisation und/oder den Phasenbereiche und/oder der Tiltwinkel und/oder den Pitch eines solchen Dielektrikums zu variieren.

In der DE 33 15 295 ist eine sehr breite allgemeine Formel für nematische Fluorphenylpyrimidine angegeben, die teilweise die hier beanspruchten Verbindungen der Formel I umfaßt. In der DE 33 15 295 sind keinerlei Hinweise für Sc-Verbindungen dieses Typs, vielmehr sollen gerade smektische Phasen unterdrückt werden. Dort sind auch keine Einzelverbindungen der hier beanspruchten Formel genannt.

Chirale Dotierstoffe für ferroelektrische Mischungen werden in der EP-A-0 278 665 beansprucht, deren breite, allgemeine Formel die erfindungsgemäßen Verbindungen umfaßt. In dort genannten Verbindungen befindet sich jedoch der laterale Halogensubstituent am Phenylring stets in meta-Position zum Pyrimidinring. Diese weisen jedoch ein positives $\Delta\epsilon$ auf und sind daher für ferroelektrische Mischungen weniger geeignet.

In der JP 63 253 075 werden ähnliche Verbindungen der Formel

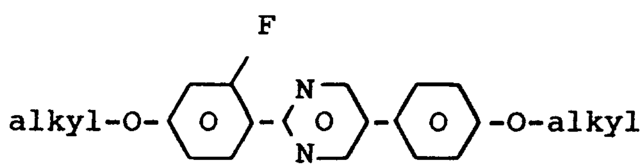

beschrieben, welche jedoch relativ hochliegende Phasenübergänge aufweisen.

In der EP-A-0 260 077 werden lateral unsubstituierte Dialkoxyphenylpyrimidine beschrieben. Der Fachmann konnte diesem Dokument keine Motivation entnehmen, diese Verbindungen mit einem lateralen Substituenten zu versehen.

Die Verbindungen der Formel I, worin einer der Reste $R^1$ oder $R^2$ eine Gruppe der Formel II bedeutet, werden von der breiten, allgemeinen Formel der WO-A-86/07055 zwar umfaßt, jedoch werden dort keine Verbindungen beschrieben, worin der Fluorsubstituent in o-Position zum Pyrimidinring steht. Weiterhin konnte der Fachmann diesem Dokument keinen Hinweis entnehmen, daß die erfindungsgemäßen Verbindungen breite Sc Phasen und eine schwach negative Anisotropie der Dielektrizitätskonstanten aufweisen.

Der Fachmann konnte somit aus dem Stand der Technik weder in einfacher Art und Weise Synthesemöglichkeiten für die beanspruchten Verbindungen entnehmen noch erkennen, daß die erfindungsgemäßen Verbindungen überwiegend breite und günstig gelegene Sc-Phasen aufweisen sowie sich durch günstige Werte für die Rotationsviskosität auszeichnen.

Gegenstand der Erfindung sind somit die Fluorphenylpyrimidine der Formel I, insbesondere der Formeln I1, I2 und I3, worin m und o jeweils 1 bis 18 bedeuten.

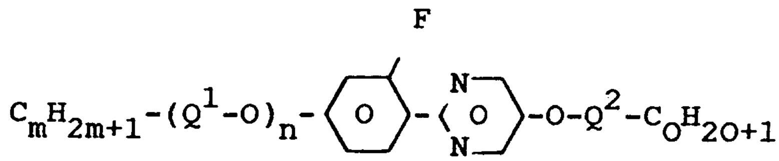

I1

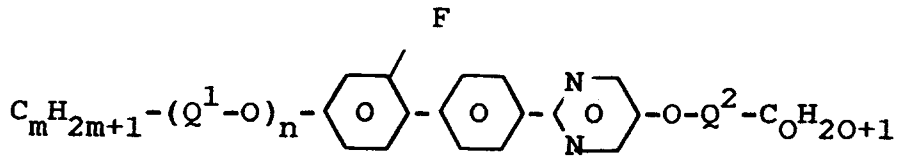

I2

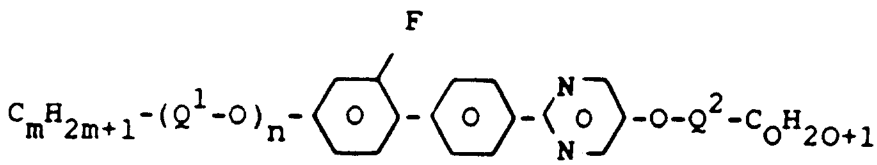

I3

Gegenstand der Erfindung sind insbesondere solche optisch aktiven Fluorphenylpyrimidine der Formel I*, worin einer der Reste $R^1$ und $R^2$ eine chirale Gruppe der Formel III,

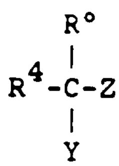

III

bedeutet,

wobei

$R^4$ eine Gruppe der Formel

$(CH_2)_r$-$Q^3$-$C_OH_{2O+1}$

worin

$Q^3$ -O-, -O-CO- oder eine Einfachbindung

r 0, 1 oder 2 und

O 1 bis 7 ist,

Y CN, Halogen oder $CH_3$,

Z eine Einfachbindung oder -$(CH_2)_p$-, worin eine $CH_2$-Gruppe durch -O-, -O-CO- oder -CO-O- ersetzt sein kann und p 1, 2, 3, 4, 5 oder 6 ist, und

R° H oder $CH_3$ bedeutet,

mit der Maßgabe, das R° von Y verschieden ist.

Gegenstand der Erfindung sind ferner ferroelektrische flüssigkristalline Phasen mit einem Gehalt an mindestens einer Verbindung der Formel I sowie Flüssigkristallanzeigelemente, insbesondere ferroelektrische elektrooptische Anzeigeelemente, die derartige Phasen enthalten.

Die erfindungsgemäßen Phasen enthalten vorzugsweise mindestens zwei, insbesondere mindestens drei Verbindungen der Formel I. Besonders bevorzugt sind erfindungsgemäße chirale getiltete smektische flüssigkristalline Phasen, deren achirale Basismischung neben Verbindungen der Formel I mindestens eine andere Komponente mit negativer oder betragsmäßig kleiner positiver dielektrischer Anisotropie enthält. Diese weiteren Komponente(n) der achiralen Basismischung können 1 bis 50 %, vorzugsweise 10 bis 25 %, der Basismischung ausmachen. Als weitere Komponenten mit betragsmäßig kleiner positiver oder negativer dielektrischer Anisotropie eignen sich Verbindungen der Formel IV, welche die Verbindungen der Teilformeln IVa bis IVi umfaßt:

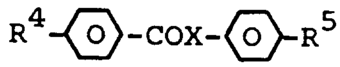
IVa

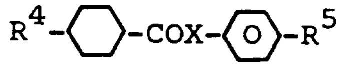
IVb

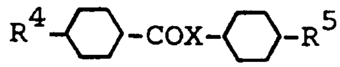
IVc

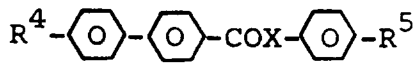
IVd

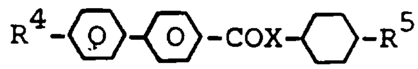
IVe

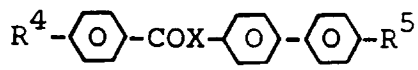
IVf

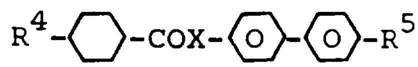
IVg

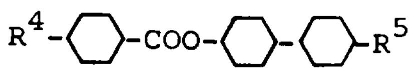
IVh

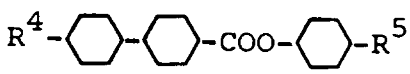
IVi

$R^4$ und $R^5$ sind jeweils vorzugsweise geradkettig Alkyl, Alkoxy, Alkanoyloxy oder Alkoxycarbonyl mit jeweils 3 bis 12 C-Atomen. X ist vorzugsweise O. In den Verbindungen der Formeln IVa, IVb, IVd, IVe, IVf und IVg kann auch eine 1,4-Phenylengruppe lateral durch Halogen oder CN, insbesondere bevorzugt durch Fluor, substituiert sein.

Besonders bevorzugt sind die Verbindungen der Teilformeln IVa, IVb, IVd und IVf, worin $R^4$ und $R^5$ jeweils geradkettiges Alkyl oder Alkoxy mit jeweils 5 bis 10 C-Atomen bedeutet.

Besonders bevorzugte Einzelverbindungen sind in der folgenden Tabelle I angegeben:

Tabelle I

| Formel | $R^4$ | $R^5$ | X |
|---|---|---|---|
| IVa | n-Decyloxy | n-Heptyloxy | O |
| IVa | n-Hexyloxy | n-Decyloxy | O |
| IVa | n-Octyloxy | n-Heptyl | O |
| IVa | n-Octyloxy | n-Pentyl | O |
| IVa | n-Decyloxy | n-Heptyl | O |
| IVa | n-Decyloxy | n-Pentyl | O |
| IVf | n-Pentyl | n-Pentyl | O |
| IVf | n-Pentyl | n-Hexyl | O |

Die Verbindungen der Teilformeln IVc, IVh und IVi eignen sich als Zusätze zur Schmelzpunktserniedrigung und werden normalerweise den Basismischungen mit nicht mehr als 5 %, vorzugsweise 1 bis 3 %, zugesetzt. $R^4$ und $R^5$ bedeuten in den Verbindungen der Teilformeln IVc, IVh und IVi vorzugsweise geradkettiges Alkyl mit 2 bis 7, vorzugsweise 3 bis 5, C-Atomen. Eine weitere zur Schmelzpunktserniedrigung in den erfindungsgemäßen Phasen geeignete Verbindungsklasse ist diejenige der Formel

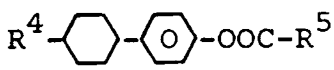

worin $R^4$ und $R^5$ die für IVc, IVh und IVi angegebene bevorzugte Bedeutung haben.

Als weitere Komponenten mit negativer dielektrischer Anisotropie eignen sich weiterhin Verbindungen enthaltend das Strukturelement A, B oder C.

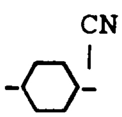

A

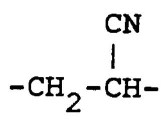

B

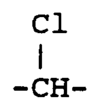

C

Bevorzugte Verbindungen dieser Art entsprechen den Formeln Va, Vb und Vc:

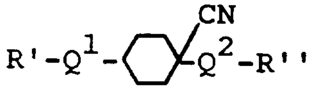

Va

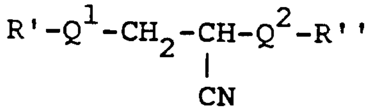

Vb

R'-$Q^3$-$Q^4$-R''' Vc

R' und R'' bedeuten jeweils vorzugsweise geradkettige Alkyl- oder Alkoxy-Gruppen mit jeweils 2 bis 10 C-Atomen. $Q^1$ und $Q^2$ bedeuten jeweils 1,4-Phenylen, trans-1,4-Cyclohexylen, 4,4'-Biphenylyl, 4-(trans-4-Cyclohexyl)-phenyl, trans,trans-4,4'-Bicyclohexyl oder eine der Gruppen $Q^1$ und $Q^2$ auch eine Einfachbin-

dung.

$Q^3$ und $Q^4$ bedeuten jeweils 1,4-Phenylen, 4,4'-Biphenylyl oder trans-1,4-Cyclohexylen. Eine der Gruppen $Q^3$ und $Q^4$ kann auch 1,4-Phenylen bedeuten, worin mindestens eine CH-Gruppe durch N ersetzt ist. R''' ist ein optisch aktiver Rest mit einem asymmetrischen Kohlenstoffatom

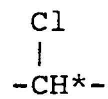 oder 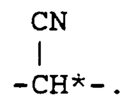.

der Struktur Besonders bevorzugte Verbindungen der Formel Vc sind diejenigen der Formel Vc':

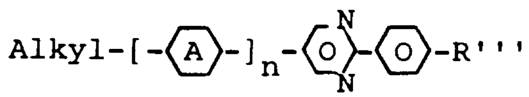 Vc'

worin A 1,4-Phenylen oder trans-1,4-Cyclohexylen und n 0 oder 1 bedeutet.

Besonders bevorzugt sind solche ferroelektrische flüssigkristalline Phasen mit einem Gehalt an mindestens einem achiralen Fluorphenylpyrimidin der Formel I1, mindestens einen achiralen Phenylpyrimidin der Formel Vc', worin R''' einen Alkyl- oder Alkoxyrest mit bis zu 18 C-Atomen bedeutet, als Basismaterial mit einer breiten $S_c$-Phase und mindestens einen chiralen Fluorphenylpyrimidin der Formel I* als optisch aktivem Dotierstoff. Weiterhin bevorzugt sind solche ferroelektrischen flüssigkristallinen Phasen, die neben den genannten Verbindungen der Formel I1, Vc' und I* mindestens ein Phenylpyridin der Formel Vd und/oder ein 2,3-Difluorphenylpyrimidin der Formel Ve und/oder ein Phenylpyrimidin der Formel Vf

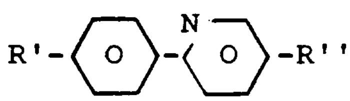 Vd

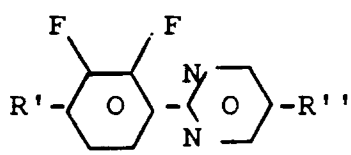 Ve

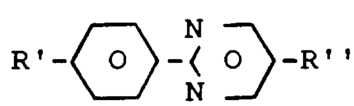 Vf

enthalten.

Die nicht-chiralen Fluorphenylpyrimidine der Formel I4

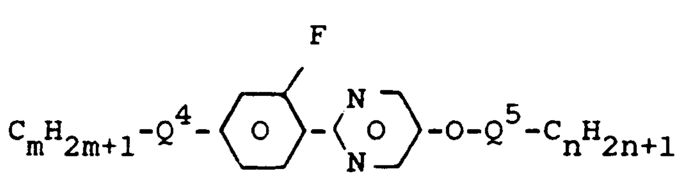 I4

worin

m und n  jeweils unabhängig voneinander 1 bis 18,

$Q^4$  -O- oder eine Einfachbindung und

$Q^5$ -CO-,

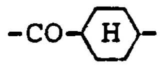, 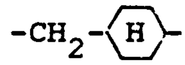

oder eine Einfachbindung bedeutet,

sind besonders als Bestandteile smektischer Basismaterialien geeignet.

Die Verbindungen der Formel I4 umfassen die nachstehend angeführten nicht-chiralen bevorzugten zweikernigen und dreikernigen Materialien der Formeln Ia bis If.

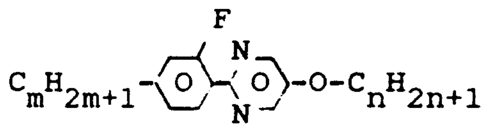 Ia

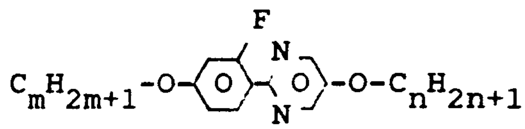 Ib

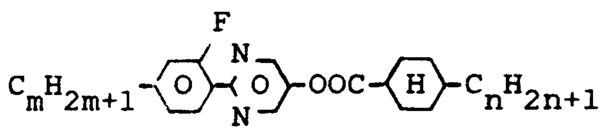 Ic

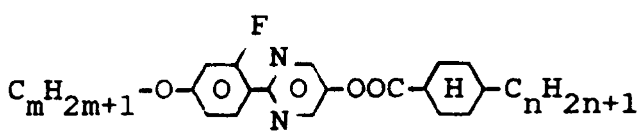 Id

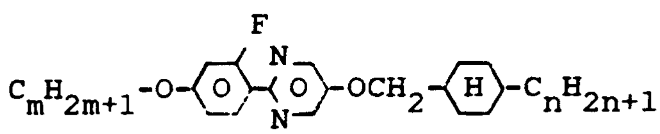 Ie

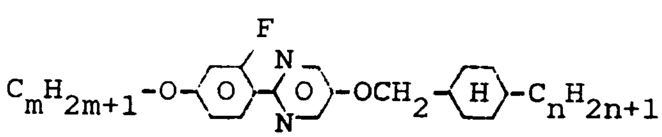 If

Die Verbindungen der Formeln I2 und I3 umfassen die nachstehend angeführten nicht-chiralen bevorzugten Verbindungen der Formeln Ig bis Ij

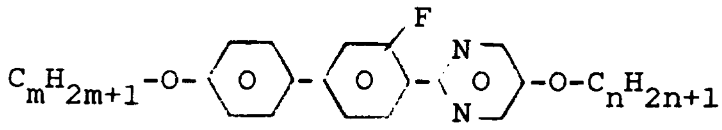 Ig

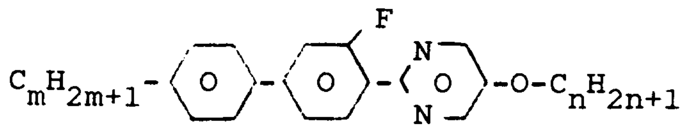 Ih

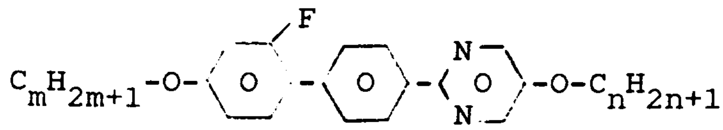 Ii

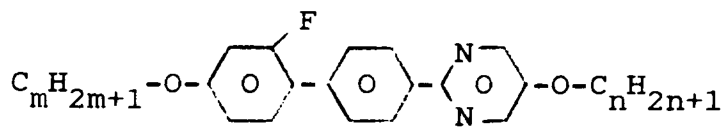 Ij

Darunter sind diejenigen der Teilformeln Ia und Ib besonders bevorzugt.

m ist vorzugsweise 5 bis 14, insbesondere 6 bis 12. n ist vorzugsweise 3 bis 12. Die Reste $C_mH_{2m+1}$ und $C_nH_{2n+1}$ sind vorzugsweise geradkettig. Verbindungen der Formel I mit relativ kurzen derartigen Resten eignen sich auch als Komponenten nematischer Phasen.

Die chiralen Fluorpyrimidine der Formel I*, worin einer der Reste $R^1$ und $R^2$ eine Gruppe der Formel III bedeutet eignen sich hervorragend als Dotierstoffe zur Induktion von Ferroelektrizität in ein smektisches Basismaterial. Sie zeichnen sich insbesondere durch eine hohe Spontanpolarisation aus.

Weiterhin destabilisieren sie die smektische Phase dieser Basismaterialien nicht. Der Rest der Formel III wird im folgenden als R* bezeichnet.

Die chiralen Verbindungen der Formel I* umfassen demnach die Verbindungen der Teilformeln Ik bis Ip mit 2 Ringen.

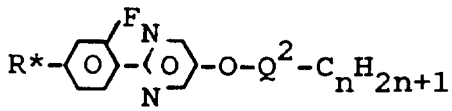

Ik

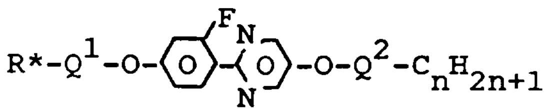

Il

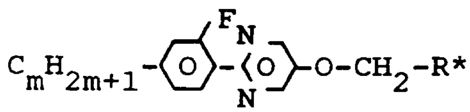

Im

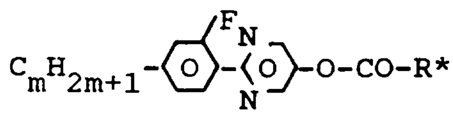

In

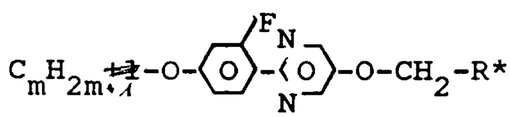

Io

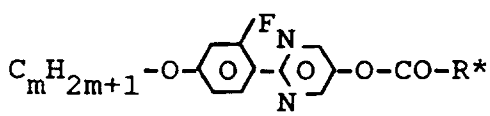

Ip

sowie die Verbindungen der Formeln Iq bis Iw mit 3 Ringen

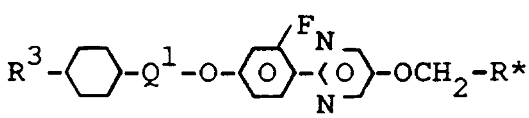

Iq

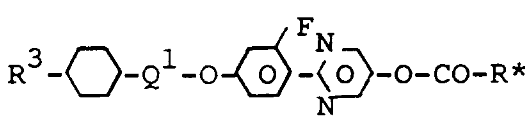

Ir

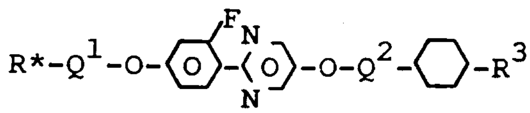

Is

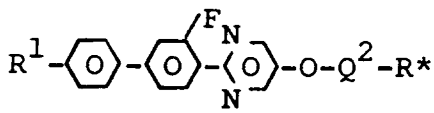

It

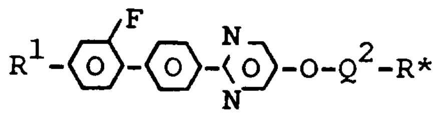

Iu

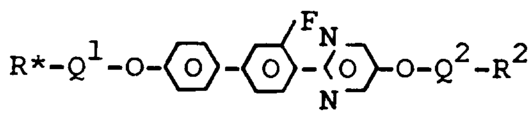

Iv

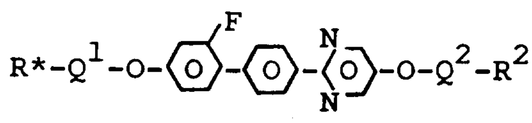

Iw

worin m und n die für die Verbindungen der Formeln Ia bis Ih angegebene bevorzugte Bedeutungen besitzen.

Insbesondere bevorzugt sind die der Teilformeln Ik und Io.

Darunter sind die chiralen Verbindungen der Formel I besonders bevorzugt, worin R* eine Gruppe der Formel IIIa bedeutet,

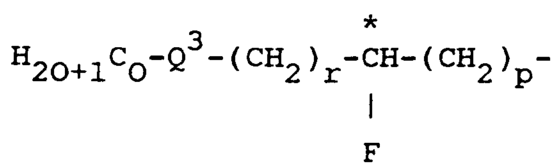

IIIa

worin O und $Q^3$ die angegebene Bedeutung besitzen, r 1 oder 2 und p 0 oder 1 ist.

Die chiralen Reste der Formel IIIa umfassen demgemäß die chiralen Monofluoralkyl-, Monofluoraoxaalkyl- und Alkanoyloxymonofluoralkylgruppe $R_f^*$ der Formeln IIIa1 bis IIIa6:

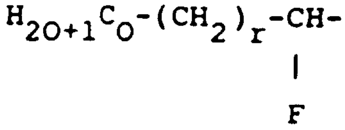

IIIa1

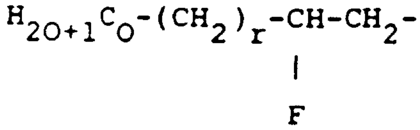

IIIa2

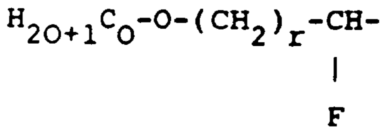

IIIa3

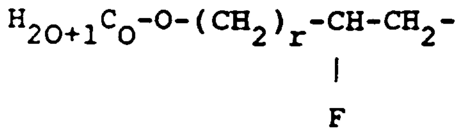

IIIa4

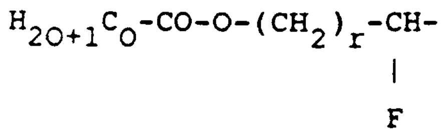

IIIa5

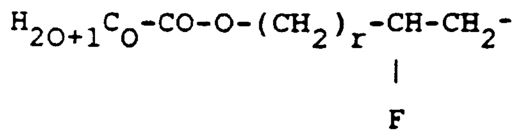

IIIa6

Von den chiralen Monofluorgruppen $R_f^*$ der Formeln IIIa1 bis IIIa6 sind diejenigen der Formeln IIIa1, IIIa3 und IIIa5 besonders bevorzugt, insbesondere diejenigen worin r 2 bedeutet.

Weiterhin bevorzugt sind die chiralen Verbindungen der Formel I, worin R* eine Gruppe der Formel IIIb

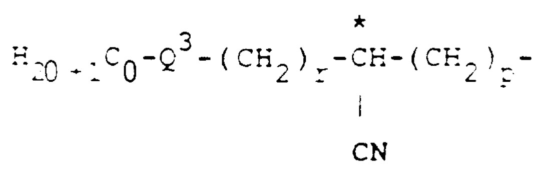

IIIb

bedeutet, worin O und $Q^3$ die angegebene Bedeutung besitzen, r 1 oder 2 ist und p 0 oder 1 ist.

Die Verbindungen der Formel I werden nach an sich bekannten Methoden, wie sie in der Literatur (z.B. in den bekannten Methoden, wie sie in der Literatur (z.B. in den Standardwerken wie Houben-Weyl, Methoden der Organischen Chemie, Georg-Thieme-Verlag, Stuttgart) beschrieben sind, hergestellt.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Die erfindungsgemäßen Verbindungen lassen sich nach folgendem Reaktionsschema einfach herstellen.

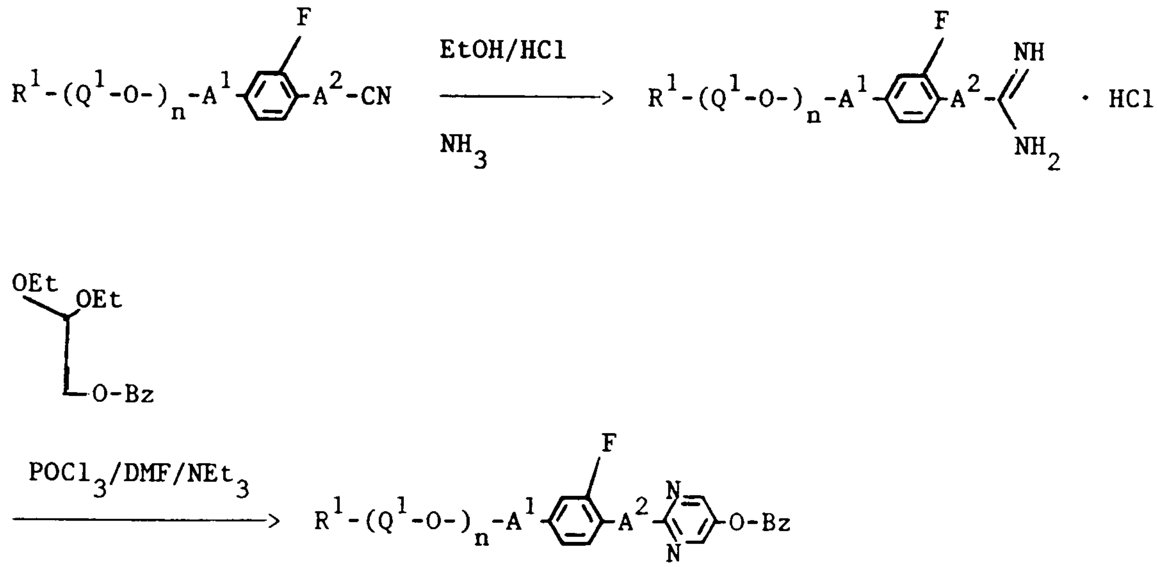


Die Benzylgruppe kann man hydrogenolytisch abspalten und dann die Hydroxygruppe erneut nach bekannten Methoden verethern oder verestern.

So können zur Herstellung von Verbindungen der Formel I2 mit r = 2 geeignete Vorstufen aus optisch aktiver Äpfelsäure nach folgendem Reaktionsschema I hergestellt werden:

Schema I

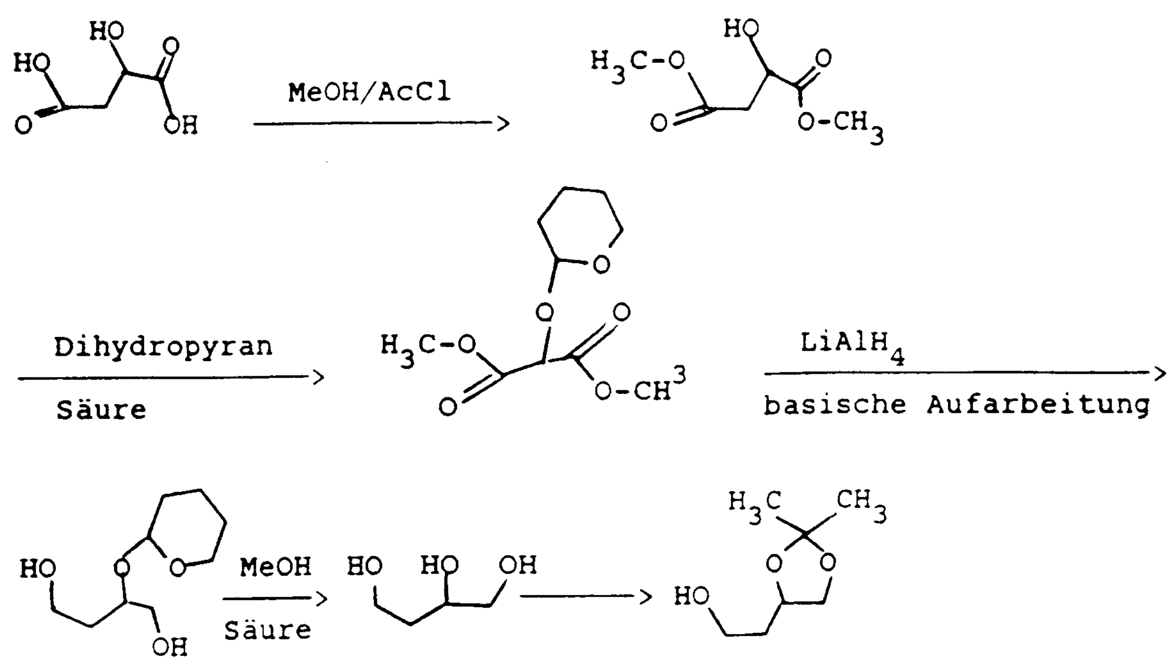


Bis zu dieser Stufe ist die Synthese von Mori et al. beschrieben worden (K. Mori, T. Takigawa and T. Matsuo, Tetrahedron 35, 933-944 (1979)).

Später haben dann Meyers and Lawson gefunden, daß die chemische Reinheit des auf diesem Weg erhaltenen Acetonids nur etwa 90 % beträgt (A.I. Meyers and J.P. Lawson, THL 23 4883-4886 (1982)).

Dessen ungeachtet kann die freie Alkoholgruppe des Acetonids nach einer der üblichen Methoden verethert werden (z.B. C.A. Brown and D. Barton, Synthesis (1974) 434 oder B.R. Jursic, Tetrahedron 44, 6677-6680 (1988)).

Der Benzylether (K. Isaac and P. Kocienski, J. Chem. Soc., Chem. Commun. (1982) 460-462) bietet sich insbesondere als Schutzgruppe an, da er spater leicht hydrogenolytisch abgespalten werden kann. Unter Standardbedingungen wird nach der Veretherung das Isopropylidenketal zum 1,2-Diol hydrolisiert und dieses dann entsprechend den Reaktionsbedingungen von Di Fabio und Misiti in das entsprechende Epoxid überführt (R. Di Fabio and D. Misiti, Gazetta Chimica Italiana 118, 209-210 (1988).

Die Behandlung des Acetonids mit HBr/Eisessig und die anschließende Umsetzung der auf diese Weise erhaltenen Bromooxalkylacetate mit K-Pentanolat liefert entsprechend der Arbeit von U. Schmidt et al. ebenfalls die gewünschten Epoxide gemäß Schema II (U. Schmidt, J. Tabiersky, F. Bartowiak and J. Wild, Angew. Chem. 92, 201-202 (1980).

Schema IIa

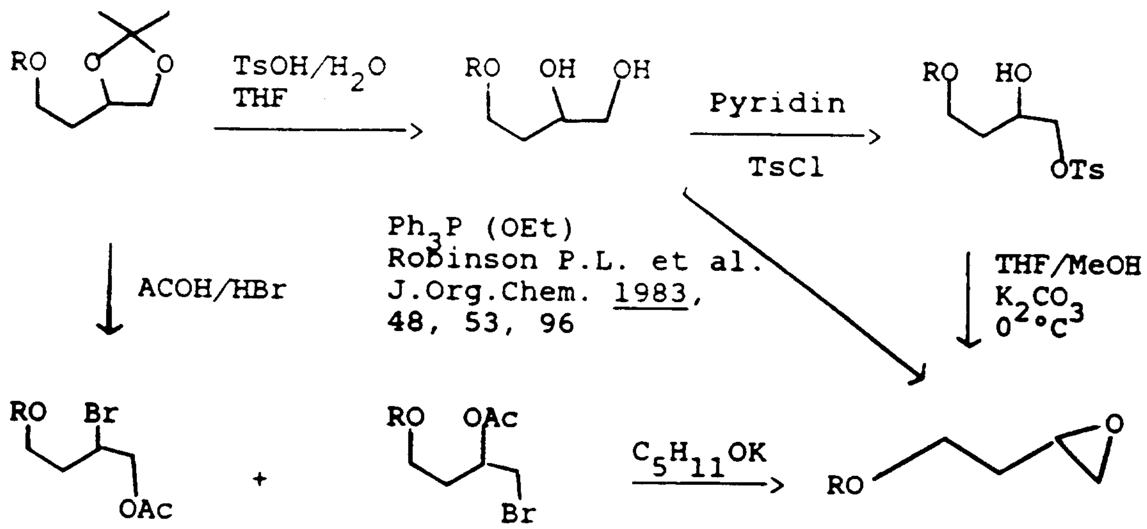


Die Umsetzung des Epoxids mit metallorganischen Verbindungen gemäß Schema IIa, bevorzugt mit Grignard-Verbindungen, ergibt unter Ringöffnung am weniger substituierten C-Atom des Epoxids mit hoher Selektivität den entsprechenden Alkohol, der mit DAST unter Standarbedingungen fluoriert wird Hydrogenolyse liefert den chiralen Alkohol, der mit Phenolen bzw. 5-Hydroxypyrimidinen verethert wird.

Schema IIb

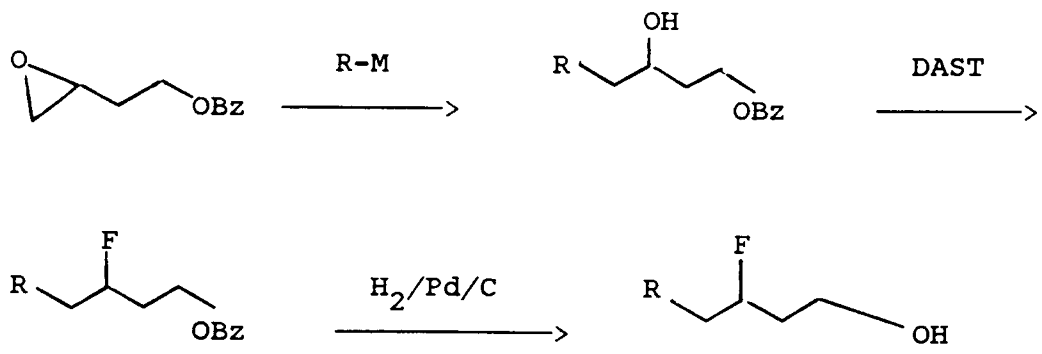


Öffnet man das Epoxid mit Pyridin/HF (H. Mongelli, F. Animati et al., Synthesis 310 (1988)), dann erhält man den entsprechenden Fluoralkohol, der anschließend in das entsprechende Tosylat überführt werden kann. Solche Tosylate eignen sich insbesondere zur Alkylierung von Phenolen und 5-Hydroxypyrimidinen

gemäß Schema III bzw. Schema IV.

Schema III

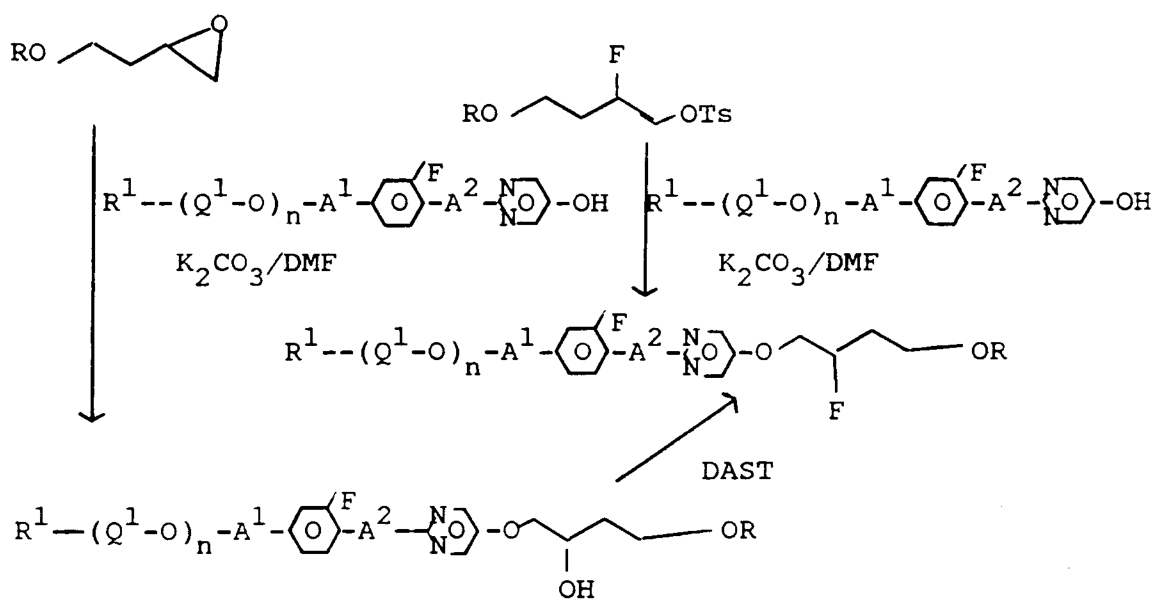


Schema IV

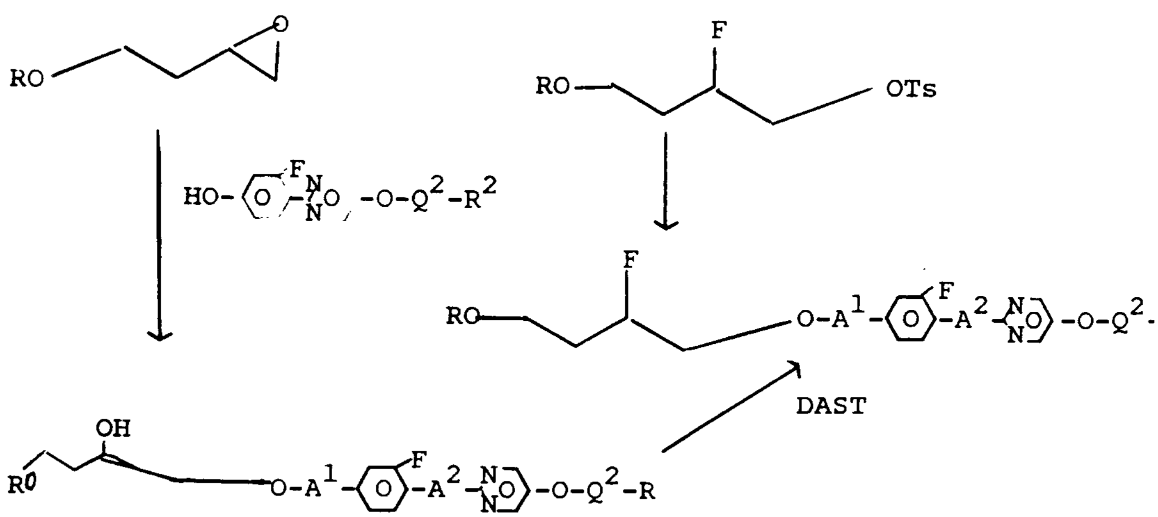


Wie obige Reaktionsschemata zeigen, kann man das Epoxid auch direkt mit Phenolen Umsetzen. Das Epoxid wird mit hoher Selektivität am weniger substituierten Kohlenstoffatom zum chiralen sekundären Alkohol geöffnet, der dann schließlich mit DAST unter Inversion in die erfindungsgemäßen Verbindungen überführt wird. Zu den üblichen Umsetzungen von Alkoholen mit DAST siehe: M. Hudlicky, Organic Reactions 35 513-637 (1987).

Die erfindungsgemäßen Verbindungen mit $Q^3$ = -O-CO- lassen sich aus den entsprechenden Benzylethern durch Hydrogenolyse und anschließende Veresterung herstellen. Folgendes Syritheseschema V beschreibt die Herstellung:

Schema V

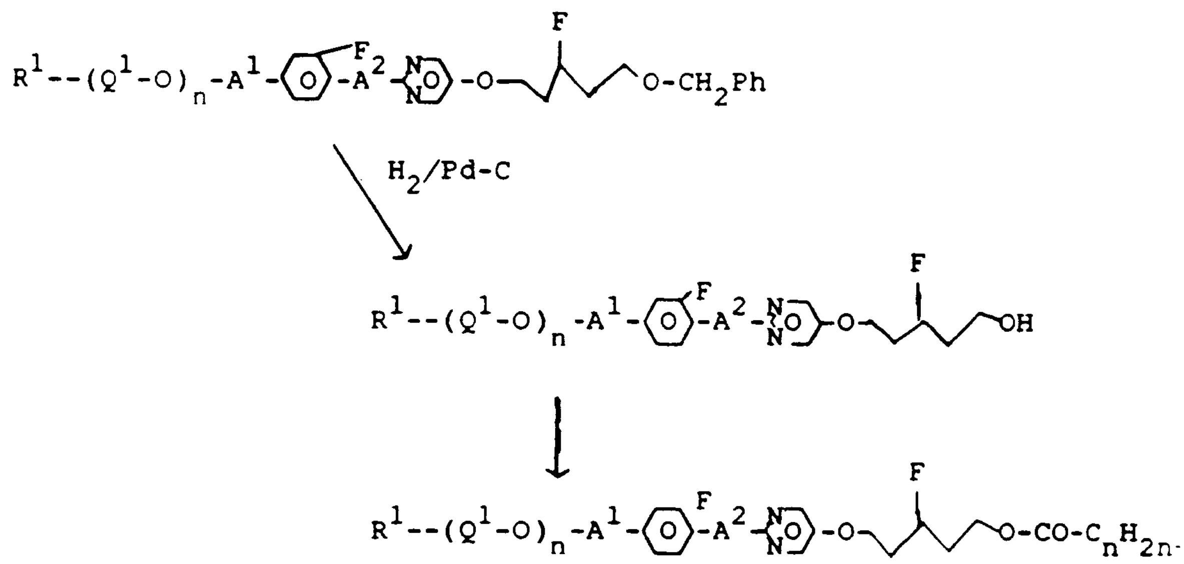


Weiterhin erhält man die Verbindungen mit $Q^3$ = -O-CO- durch Oxidation der entsprechenden Fluoralkohole und anschließende Veresterung mit mesogenen Phenolen gemäß Schema VI:

Schema VI

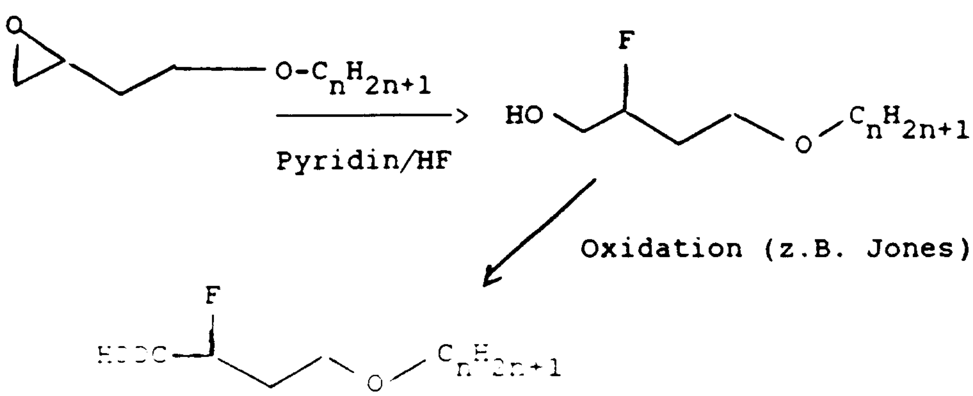


Wenn bei der Oxidation Racemisierung auftritt, kann man die optisch aktiven Fluorsäuren durch Racematspaltung nach Helmchen gewinnen (Angew. Chem. 91, 65 (1979)).

CH-azide Verbindungen, wie beispielsweise 2-Fluor-Tolunitril öffnen in Gegenwart geeigneter Basen ebenfalls das Epoxid zum optisch aktiven sekundären Alkohol, der dann mit DAST unter Inversion fluoriert wird. Bevorzugt Reaktionswege können der folgenden Schema VII entnommen werden.

Schema VII

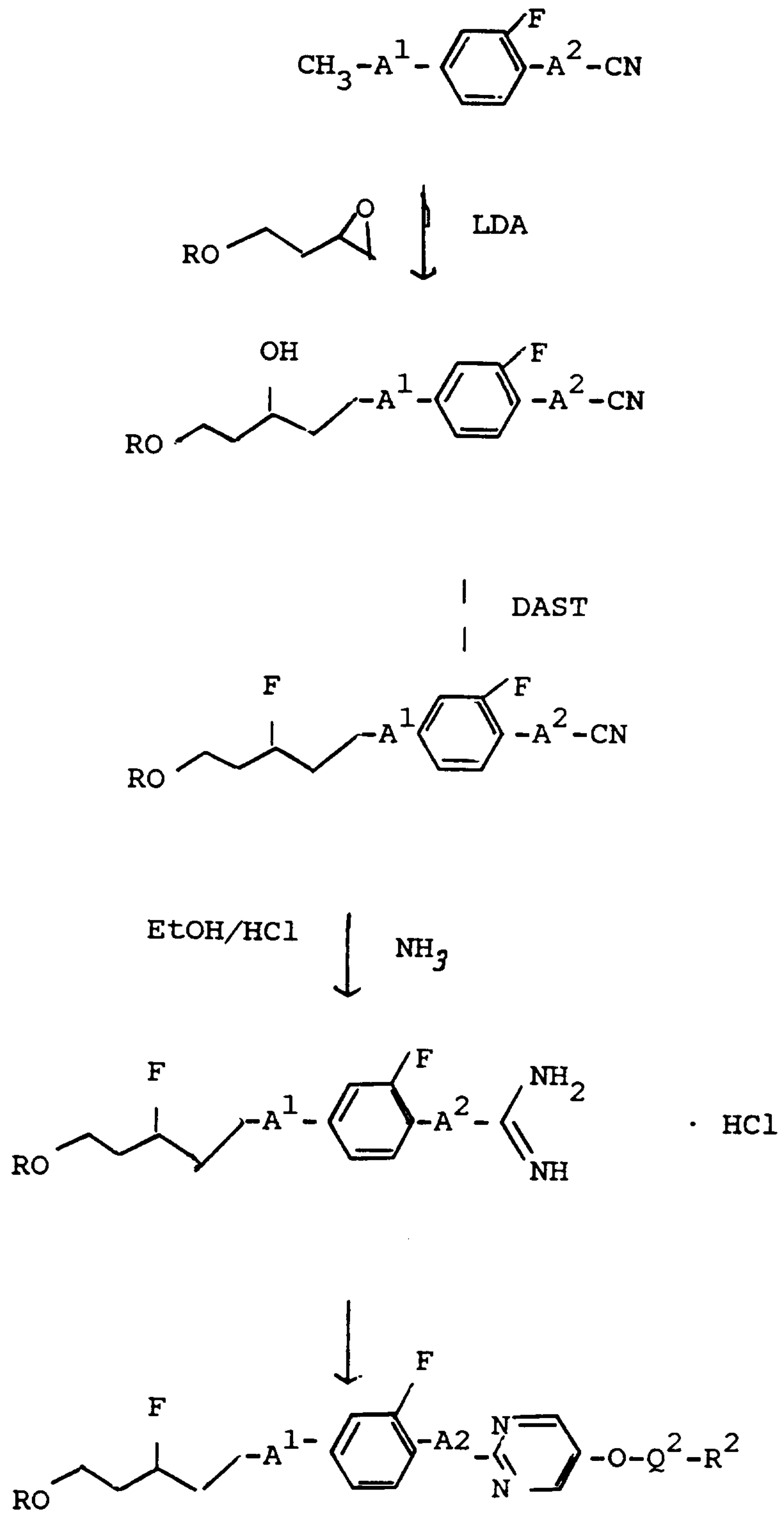


Die Verbindungen der Formel I mit r = 1 können analog hergestellt werden unter Einsatz der bekannten Epoxide der Formel

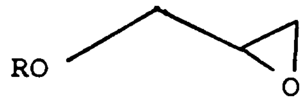

oder der aus diesen nach üblichen Verfahren erhältlichen Fluoralkohole der Formel

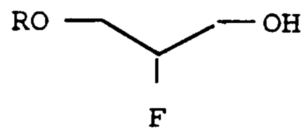

Die Verbindungen der Formel I eignen sich auch als Komponenten nematischer flüssigkristalliner Phasen, z.B. zur Vermeidung von reverse twist.

Diese erfindungsgemäßen flüssigkristallinen Phasen bestehen aus 2 bis 25, vorzugsweise 3 bis 15 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexylpyridazine sowie deren N-Oxide, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel I' charakterisieren,

R'-L-G-E-R''    I''

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

G -CH = CH- -N(O) = N-
-CH = CY- -CH = N(O)-
-C≡C- $-CH_2-CH_2-$
-CO-O- $-CH_2-O-$
-CO-S- $-CH_2-S-$
-CH = N- -COO-Phe-COO-
oder eine C-C-Einfachbindung,
Y Halogen, vorzugsweise Chlor, oder -CN, und

R' und R'' Alkyl, Alkoxy, Alkanoyloxy, Alkoxycarbonyl oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind R' und R'' voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden erhältlich.

Die erfindungsgemäßen Phasen enthalten etwa 0,1 bis 99, vorzugsweise 10 bis 95 %, einer oder mehrerer Verbindungen der Formel I. Weiterhin bevorzugt sind erfindungsgemäße flüssigkristalline Phasen, enthaltend 0,1-40, vorzugsweise 0,5-30 % einer oder mehrerer Verbindungen der Formel I.

Die Herstellung der erfindinngsgemäßen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol. Cryst.Liq.Cryst. Band 24, Seiten 249-258 (1973)) zur Verbesserung der Leitfähigkeit, pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Derartige Substanzen sind z.B. in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Weiterhin bedeuten:

| | |
|---|---|
| K | kristallin |
| N | nematisch |
| S | smektisch |
| I | isotrop |

Die zwischen diesen Symbolen stehenden Zahlen geben die jeweilige Phasenübergangstemperatur in °C an. Ferner werden folgende Abkürzungen verwendet:

| | |
|---|---|
| DAST | Diethylaminoschwefeltrifluorid |
| DMF | Dimethylformamid |
| n-BuLi | n-Butyllithium |

Beispiel 1

Zu 0,15 mol $POCl_3$ wird unter Kühlung 0,185 mol DMF gegeben. Nach 15 Minuten gibt man eine Lösung von 0,1 mol Benzyloxyacetaldehyddiethylacetal in 50 ml DMF zu und erwärmt anschließend auf 50 °C. Nach 12 Stunden wird das Reaktionsgemisch auf Raumtemperatur abgekühlt und 0,1 mol 4-Heptyloxy-2-fluorbenzamidinhydrochlorid zugegeben. Die Temperatur steigt dabei auf etwa 40 °C an. Das Reaktionsgemisch wird 30 Minuten gerührt, danach gibt man 110 ml Triethylamin zu. Die Temperatur steigt dabei auf etwa 70 °C an, das Reaktionsgemisch wird zähflüssig. Zur besseren Rührbarkeit kann man das Reaktionsgemisch mit DMF verdünnen. Danach destilliert man das Triethylamin ab, läßt den Rückstand auf etwa 100 °C abkühlen, gibt dann 500 ml Wasser zu und säuert mit konz. HCl an. Der dabei entstandene Niederschlag wird abgesaugt, gründlich mit Wasser gewaschen und im Vakuum getrocknet. Die so erhaltene Benzyloxyverbindung wird in THF aufgenommen und bei Raumtemperatur drucklos mit einem Pd-Katalysator (Pd-C-5 % E101RW) hydriert. Nach dem Entfernen des Katalysators wird die Lösung im Vakuum eingedampft, das Hydroxypyrimidin in Methylethylketon aufgenommen und in Gegenwart einer äquivalenten Menge an getrocknetem Kaliumcarbonat und Bromnonan 12 Stunden am Rückfluß gekocht. Die übliche Aufarbeitung liefert 2-(4-Heptyloxy-2-fluor-phenyl)-5-nonyloxypyrimidin mit folgenden Phasenübergängen: K 39 $S_c$ 60 N 73.1, $\Delta\epsilon$ = -0,1.

Analog werden folgende Verbindungen der Formel I4 hergestellt:

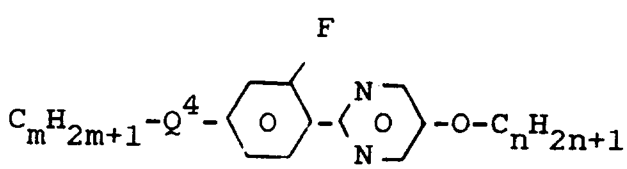

| m | $Q^4$ | n | K | $S_C$ | N | I | Δε |
|---|---|---|---|---|---|---|---|
| 7 | 0 | 8 | 39 | 49 | 73,6 | | -0,1 |
| 7 | 0 | 10 | 41 | 69 | 76,1 | | -0,1 |
| 7 | 0 | 11 | 56 | 73 | 75,3 | | -0,1 |
| 7 | 0 | 12 | 47 | 77 | 77,6 | | 0,0 |
| 8 | 0 | 8 | 42 | 52 | 77,3 | | -0,1 |
| 8 | 0 | 9 | 39 | 62 | 75,4 | | -0,1 |
| 8 | 0 | 10 | 40 | 72 | 78,2 | | -0,1 |
| 8 | 0 | 11 | 49 | 76 | 77,9 | | 0,0 |
| 8 | 0 | 12 | 51 | 80 | - | | 0,0 |
| 10 | 0 | 8 | 48 | 58 | 77,2 | | 0,0 |
| 10 | 0 | 9 | 45 | 67 | 76,4 | | +0,1 |
| 10 | 0 | 10 | 45 | 75 | 79,2 | | +0,1 |
| 10 | 0 | 11 | 50 | 79 | 79,6 | | +0,1 |
| 10 | 0 | 12 | 54 | 82,6 | - | | 0,0 |
| 12 | 0 | 8 | 51 | 62 | 76 | | +0,1 |
| 12 | 0 | 9 | 49 | 69 | 75,9 | | 0,0 |
| 12 | 0 | 10 | 52 | 77 | 79,1 | | +0,1 |
| 12 | 0 | 11 | 58 | 80 | - | | +0,1 |
| 12 | 0 | 12 | 61 | 84 | - | | +0,5 |

| | | | | |
|---|---|---|---|---|
| 8 | 0 | $OCH_2$- 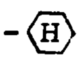 - | 3 | |
| 8 | 0 | $OCH_2$- 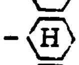 - | 4 | |
| 8 | 0 | $OCH_2$- 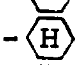 - | 5 | |
| 8 | 0 | $OCH_2$- 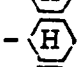 - | 7 | |
| 7 | 0 | $OCH_2$- 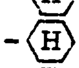 - | 3 | |
| 7 | 0 | $OCH_2$- 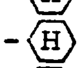 - | 4 | K 92 $S_A$ 141 N 152 9 I, |
| 7 | 0 | $OCH_2$- 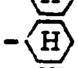 - | 5 | |
| 7 | 0 | $OCH_2$- 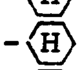 - | 7 | |
| 7 | 0 | $OCH_2$- 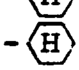 - | 8 | K 99 $S_A$ 150 I |

Analog erhält man aus 2-(4'-Alkyl-2-fluor-biphenyl-4-yl)-5-hydroxypyrimidinen (hergestellt aus 4'-Alkyloxy-2-fluorbiphenyl-4-yl-carbamidinen entsprechend Beispiel 1) die Verbindungen der Formel I2.

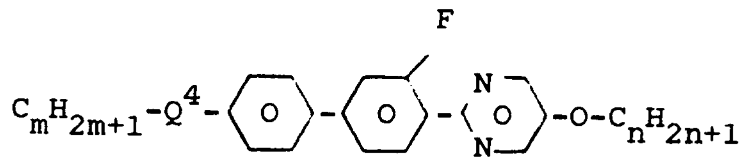

| m | $Q^4$ | n |
|---|---|---|
| 8 | 0 | 7 |
| 8 | 0 | 8 |
| 5 | - | 7 |
| 9 | 0 | 6 |
| 9 | 0 | 7 |
| 9 | 0 | 8 |
| 9 | 0 | 9 |
| 4 | 0 | 5 |
| 3 | - | 7 |

Analog erhält man aus 2-(4′-Alkyloxy-2'-fluorbiphenyl-4-yl)-5-hydroxypyrimidinen (hergestellt aus 4'-Alkoxy-2'-fluorbiphenyl-4-yl-carbamidinen entsprechend Beispiel 1) die Verbindungen der Formel I3

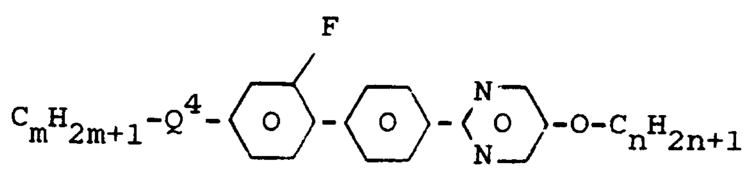

| m | $Q^4$ | n |
|---|---|---|
| 8 | 0 | 7 |
| 8 | 0 | 8 |
| 6 | 0 | 6 |
| 5 | - | 3 |
| 5 | - | 7 |
| 9 | 0 | 6 |
| 9 | 0 | 7 |
| 9 | 0 | 8 |
| 9 | 0 | 9 |
| 4 | 0 | 5 |
| 3 | - | 7 |
| 11 | 0 | 6 |
| 6 | 0 | 11 |

Beispiel 2

0,1 mol 2-(4-Heptyloxy-2-fluorphenyl)-5-hydroxypyrimidin (hergestellt aus 4-Heptyloxy-2-fluorbenzamidin entsprechend Beispiel 1) wird zusammen mit 0,12 mol Pyridin in Toluol gelöst und dann bei Raumtemperatur 0,1 mol Nonansäurechlorid zugegeben. Dann wird 12 Stunden gerührt und wie üblich aufgearbeitet. Man erhält 2-(4-Heptyloxy-2-fluorphenyl)-5-nonanoyloxy-pyrimidin, K 65 I, $\Delta\epsilon$ = -0,2.

Analog erhält man folgende Ester der Formel I:

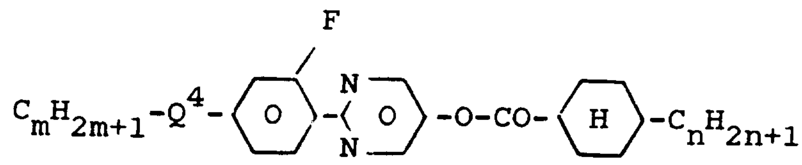

| m | $Q^4$ | n | |
|---|---|---|---|
| 8 | O | 3 | |
| 8 | O | 5 | |
| 8 | O | 7 | |
| 8 | O | 9 | |
| 8 | O | 10 | |
| 8 | O | 11 | |
| 8 | - | 3 | |
| 8 | O | 5 | |
| 10 | - | 3 | |
| 7 | O | 7, | K 70 $S_C$ 133 $S_A$ 144 N 178,2 I, |
| 7 | O | 11 | $\Delta\varepsilon$ = -0,2 |
| 7 | - | 3 | |

Beispiel 3

Man kühlt eine Lösung von 0,1 mol 5-Heptyloxy-2-(4-(2-hydroxy-5-oxaoctyloxy)-2-fluorphenyl)-pyrimidin (hergestellt durch Erhitzen von optisch aktivem 1,2-Epoxy-5-oxaoctan, erhältlich aus Äpfelsäure, mit 5-Heptyloxy-2-(4-hydroxy-2-fluorphenyl)pyrimidin in Gegenwart von trockenem Kaliumcarbonat und Methylethylketon als Lösungsmittel) in Methylenchlorid auf -40 °C und gibt dazu unter Feuchtigkeitsausschluß tropfenweise 0,11 mol DAST. Anschließend rührt man das Reaktionsgemisch unter langsamem Erwärmen auf Raumtemperatur 12 Stunden. Dann hydrolysiert man unter Eiskühlung und wäscht das Reaktionsgemisch mit verdünnter Natronlauge und mehrfach mit Wasser. Nach dem Trocknen über Magnesiumsulfat wird das Lösungsmittel am Rotationsverdampfer entfernt und das Rohprodukt chromatographisch und durch Kristallisation gereinigt. Man erhält optisch aktives 5-Heptyloxy-2-(4-(2-fluor-5-oxaoctyloxy)-2-fluorphenyl)-pyrimidin.

Analog werden folgende Verbindungen der Formel I1 hergestellt:

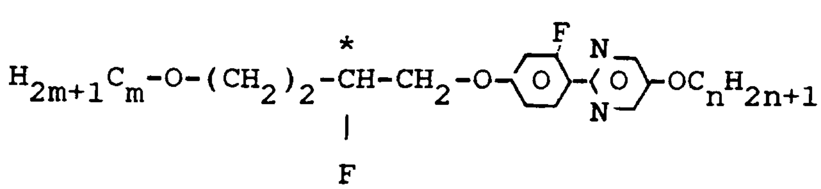

| m | n |
|---|---|
| 2 | 7 |
| 4 | 7 |
| 5 | 7 |
| 2 | 8 |
| 3 | 8 |
| 4 | 8 |
| 5 | 8 |
| 3 | 9 |
| 3 | 10 |

Beispiel 4

Aus 0,1 mol 2-(4-Octyloxy-2-fluorphenyl)-5-hydroxypyrimidin (hergestellt entsprechend Beispiel 1) und 0,1 mol optisch aktiven 1,2-Epoxy-5-octan erhält man in Analogie zu Beispiel 3 2-(4-Octyloxy-2-fluorphenyl)-5-(2-fluor-5-oxyoctyloxy)-pyrimidin.

Analog werden folgende Verbindungen der Formel I1 hergestellt:

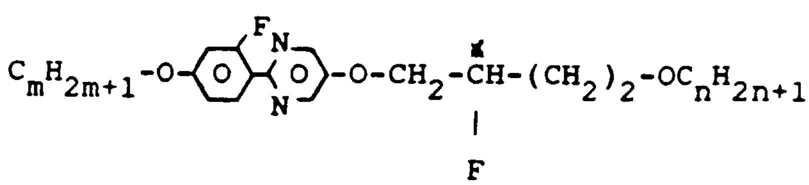

| m | n |
|---|---|
| 7 | 3 |
| 9 | 3 |
| 11 | 3 |

Beispiel 5

Zu einer Lösung von 0,15 mol 2-(4-Decyloxy-2-fluorphenyl)-5-hydroxypyrimidin (hergestellt entsprechend Beispiel 1), 0,17 mol L(-)-Ethyllactat und 0,15 mol Triphenylphosphin in 400 ml THF gibt man 0,17 mol Azodicarbonsäurediethylester (DEAD) gelöst in THF. Dabei soll eine Reaktionstemperatur von 50 °C nicht überschritten werden. Man rührt 1 Stunde bei 50 °C und dann über Nacht bei Raumtemperatur. Anschließend destilliert man das Lösungsmittel ab, löst den Rückstand in heißem Toluol und läßt dann langsam abkühlen. Das ausgefallene Triphenylphosphinoxid wird abgesaugt, das Filtrat eingeengt und der Rückstand chromatographisch gereinigt. Man erhält 2-[2-(4-Decyloxy-2-fluorphenyl)-pyrimidin-5-oxy]-propionsäureethylester.

Beispiel 6

Optisch aktiver Milchsäurebenzylester wird mittels Diethylazodicarboxylat (DEAD)/Triphenylphosphin mit 2-(4-Octyloxy-2-fluorphenyl)-5-hydroxypyrimidin (hergestellt entsprechend Beispiel 1) verethert und anschließend die Benzylgruppe hydrogenolytisch abgespalten. Die so gewonnene Säure wird wie üblich in das Nitril überführt (Oxalylchlorid, Ammoniak, Thionylchlorid). Man erhält optisch aktives 2-(4-Octyloxy-2-fluorphenyl)-5-(1-cyanethoxy)-pyrimidin.

Beispiel 7

Zu einem Gemisch aus 0,1 mol 2-(4-Octyl-2-fluorphenyl)-5-hydroxypyrimidin(hergestellt entsprechend Beispiel 1), 0,1 mol optisch aktiver 2-Chlor-3-methylbuttersäure (hergestellt aus Valin) und einer katalytischen Menge 4-(N,N-Dimethylamino)pyridin in 250 ml Methylenchlorid gibt man bei 0 °C eine Lösung von 0,1 mol DCC in Methylenchlorid. Anschließend läßt man 12 Stunden bei Raumtemperatur ruhen, saugt dann den Niederschlag ab, arbeitet das Filtrat wie üblich auf und erhält 2-Chlor-3-methylbuttersäure-[2-(4-Octyl-2-fluorphenyl)-pyrimidin-5-ylester]

Die folgenden Beispiele betreffen ferroelektrische flüssigkristalline Medien

Beispiel A

Man stellt ein flüssigkristallines Medium her bestehend aus:

5,8 % (2-Hexyloxyphenyl)-5-heptylpyrimidin
5,8 % (2-Octyloxyphenyl)-5-heptylpyrimidin
5,8 % (2-Decyloxyphenyl)-5-heptylpyrimidin
1,1 % (2-p-Octyloxyphenyl)-5-octylpyrimidin
1,1 % (2-p-Nonyloxyphenyl)-5-octylpyrimidin
1,1 % (2-p-Decyloxyphenyl)-5-octylpyrimidin
7,0 % 2-(4-Decyloxy-2-fluorphenyl)-5-dodecyloxypyrimidin
14,1 % 2-(4-Heptyloxy-2-fluorphenyl)-5-dodecyloxypyrimidin
14,1 % 2-(4-Decyloxy-2-fluorphenyl)-5-undecyloxypyrimidin
5,0 % 2-(4-Octyloxy-2,3-difluorphenyl)-5-nonylpyrimidin
5,0 % 2-(4-Heptyloxy-2,3-difluorphenyl)-5-nonylpyrimidin
5,0 % 2-(4-Nonyloxy-2,3-difluorphenyl)-5-nonylpyrimidin
18,0 % 2-(4-Hexyloxyphenyl)-5-hexyloxypyrimidin und
10 % optisch aktivem 2-[4-(2-Fluoroctyloxy)-2,3-difluorphenyl]-5-heptylpyrimidin.

Dieses Medium weist folgende physikalische Eigenschaften auf:

| | |
|---|---|
| Phasenübergänge: | K < -20 $S_c$* 59 Ch 72 I |
| Spontanpolarisation (20 °C): | -13,1 nC $cm^{-2}$ |
| Schaltzeit (20 °C): | 125 $\mu$s |

Beispiel B

Man stellt ein flüssigkristallines Medium her bestehend aus:

5,8 % 2-(p-Hexyloxyphenyl)-5-heptylpyrimidin
5,8 % 2-(p-Octyloxyphenyl)-5-heptylpyrimidin
5,8 % 2-(p-Decyloxyphenyl)-5-heptylpyrimidin
1,1 % 2-(p-Octyloxyphenyl)-5-octylpyrimidin
1,1 % 2-(p-Nonyloxyphenyl)-5-octylpyrimidin
2,2 % 2-(p-Decyloxyphenyl)-5-octylpyrimidin
5,0 % 2-(4-Decyloxy-2-fluorphenyl)-5-dodecyloxypyrimidin
10,1 % 2-(4-Heptyloxy-2-fluorphenyl)-5-dodecyloxypyrimidin
10,1 % 2-(4-Decyloxy-2-fluorphenyl)-5-undecyloxypyrimidin
10 % 2-(4-Octyloxy-2,3-difluorphenyl)-5-heptylpyrimidin
5 % 2-(4-Octyloxy-2,3-difluorphenyl)-5-nonylpyrimidin
5 % 2-(4-Heptyloxy-2,3-difluorphenyl)-5-nonylpyrimidin
5 % 2-(4-Nonyloxy-2,3-difluorphenyl)-5-nonylpyrimidin
18 % 2-(p-Hexyloxyphenyl)-5-hexyloxypyrimidin und
10 % optisch aktivem 2-[4-(2-Fluoroctyloxy)-2-fluorphenyl]-5-heptyloxypyrimidin

Dieses Medium weist einen $S_c$*-Phasenbereich von über 60 °C und eine hohe Spontanpolarisation auf.

Beispiel C

Man stellt ein flüssigkristallines Medium her bestehend aus:

5,8 % 2-(p-Hexyloxyphenyl)-5-heptylpyrimidin
5,8 % 2-(p-Octyloxyphenyl)-5-heptylpyrimidin
5,8 % 2-(p-Decyloxyphenyl)-5-heptylpyrimidin

1,1 % 2-(p-Octyloxyphenyl)-5-octylpyrimidin
1,1 % 2-(p-Nonyloxyphenyl)-5-octylpyrimidin
2,2 % 2-(p-Decyloxyphenyl)-5-octylpyrimidin
5,0 % 2-(4-Decyloxy-2-fluorphenyl)-5-dodecyloxypyrimidin
10,1 % 2-(4-Heptyloxy-2-fluorphenyl)-5-dodecyloxypyrimidin
10,1 % 2-(4-Decyloxy-2-fluorphenyl)-5-undecyloxypyrimidin
10 % 2-(4-Octyloxy-2,3-difluorphenyl)-5-heptylpyrimidin
5 % 2-(4-Octyloxy-2,3-difluorphenyl)-5-nonylpyrimidin
5 % 2-(4-Heptyloxy-2,3-difluorphenyl)-5-nonylpyrimidin
5 % 2-(4-Nonyloxy-2,3-difluorphenyl)-5-nonylpyrimidin
18 % 2-(p-Hexyloxyphenyl)-5-hexyloxypyrimidin und
10 % optisch aktivem 2-(4-Octyloxy-2-fluorphenyl)-5-(1-cyanethoxy)-pyrimidin
Dieses Medium weist einen $S_c$*-Phasenbereich von über 50 °C und eine hohe Spontanpolarisation auf.

Beispiel D

Man stellt ein flüssigkristallines Medium her bestehend aus:
5,8 % 2-(p-Hexyloxyphenyl)-5-heptylpyrimidin
5,8 % 2-(p-Octyloxyphenyl)-5-heptylpyrimidin
5,8 % 2-(p-Decyloxyphenyl)-5-heptylpyrimidin
1,1 % 2-(p-Octyloxyphenyl)-5-octylpyrimidin
1,1 % 2-(p-Nonyloxyphenyl)-5-octylpyrimidin
2,2 % 2-(p-Decyloxyphenyl)-5-octylpyrimidin
5,0 % 2-(4-Decyloxy-2-fluorphenyl)-5-dodecyloxypyrimidin
10,1 % 2-(4-Heptyloxy-2-fluorphenyl)-5-dodecyloxypyrimidin
10,1 % 2-(4-Decyloxy-2-fluorphenyl)-5-undecyloxypyrimidin
5,0 % 2-(4-Octyloxy-2-fluorphenyl)-5-dodecyloxypyrimidin
10 % 2-(4-Octyloxy-2,3-difluorphenyl)-5-heptylpyrimidin
5 % 2-(4-Octyloxy-2,3-difluorphenyl)-5-nonylpyrimidin
5 % 2-(4-Heptyloxy-2,3-difluorphenyl)-5-nonylpyrimidin
18 % 2-(p-Hexyloxyphenyl)-5-hexyloxypyrimidin und
10 % optisch aktivem 2-[4-(2-Fluoroctyloxy)-2-fluorphenyl]-5-heptyloxypyrimidin
Dieses Medium weist einen breiten $S_c$*-Phasenbereich und eine hohe Spontanpolarisation auf.

Beispiel E

Man stellt ein flüssigkristallines Medium her bestehend aus:
5,8 % 2-(p-Hexyloxyphenyl)-5-heptylpyrimidin
5,8 % 2-(p-Octyloxyphenyl)-5-heptylpyrimidin
5,8 % 2-(p-Decyloxyphenyl)-5-heptylpyrimidin
1,1 % 2-(p-Octyloxyphenyl)-5-octylpyrimidin
1,1 % 2-(p-Nonyloxyphenyl)-5-octylpyrimidin
2,2 % 2-(p-Decyloxyphenyl)-5-octylpyrimidin
5,0 % 2-(4-Decyloxy-2-fluorphenyl)-5-dodecyloxypyrimidin
10,1 % 2-(4-Heptyloxy-2-fluorphenyl)-5-dodecyloxypyrimidin
10,1 % 2-(4-Decyloxy-2-fluorphenyl)-5-undecyloxypyrimidin
5,0 % 2-(4-Octyloxy-2-fluorphenyl)-5-dodecyloxypyrimidin
10 % 2-(4-Octyloxy-2,3-difluorphenyl)-5-heptylpyrimidin
5 % 2-(4-Octyloxy-2,3-difluorphenyl)-5-nonylpyrimidin
5 % 2-(4-Nonyloxy-2,3-difluorphenyl)-5-nonylpyrimidin
18 % 2-(p-Hexyloxyphenyl)-5-hexyloxypyrimidin und
18 % optisch aktivem 2-[4-(2-Fluoroctyloxy)-2-fluorphenyl]-5-heptyloxypyrimidin
2 % optisch aktivem 2-[4-(2-Fluoroctyloxy)-2,3-difluorphenyl]-5-heptylpyrimidin
Dieses Medium weist einen breiten $S_c$*-Phasenbereich und eine hohe Spontanpolarisation auf.

**Patentansprüche**

1. Fluorphenylpyrimidine der Formel I,

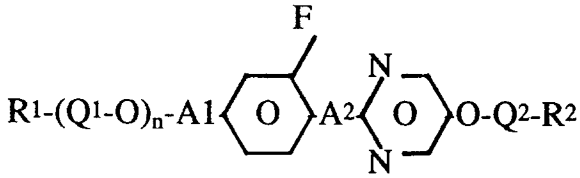

wobei

| | |
|---|---|
| $Q^1$ und $Q^2$ | jeweils unabhängig voneinander CO oder $CH_2$, |
| n | 0 oder 1, |
| $A^1$ and $A^2$ | jeweils unabhängig voneinander 1,4-Phenylen oder eine Einfachbindung, und |
| $R^1$ und $R^2$ | jeweils unabhängig voneinander ein unsubstituierter oder mit CN oder mit mindestens einem Halogen substituierter Alkyl- oder Alkenylrest mit bis zu 18 C-Atomen, worin eine oder mehrere $CH_2$-Gruppen ersetzt sein können durch einen Rest ausgewählt aus der Gruppe -O-, -CO-O-, -O-CO- oder -C≡C-wobei zwei Sauerstoffatome nicht benachbart sind, |

bedeuten, und im Falle n = 1
einer der Reste $R^1$ und $R^2$ auch eine Gruppe der Formel II bedeuten kann,

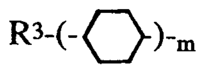

worin

| | |
|---|---|
| $R^3$ | eine unsubstituierte oder mit CN oder mit mindestens einem Halogen substituierte Alkyl-, Alkenyl- oder Alkoxygruppe mit bis zu 18 C-Atomen bedeutet, und |
| m | 1 oder 2 ist. |

2. Fluorphenylpyrimidine der Formel I, wobei mindestens einer der Reste $R^1$ und $R^2$ eine Gruppe der Formel III,

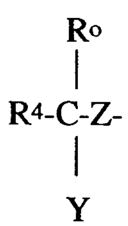

III

wobei

| | |
|---|---|
| $R^4$ | eine Gruppe der Formel $-(CH_2)_r-Q_3-C_oH_{2o+1}$, |

worin

| | |
|---|---|
| r | 0, 1 oder 2, |
| o | 1 bis 7, und |
| $Q^3$ | -O-, -O-CO-oder eine Einfachbindung |

bedeutet,

| | |
|---|---|
| Y | CN, Halogen oder $CH_3$, |
| Z | eine Einfachbindung oder $-(CH_2)_p-$, worin eine $CH_2$-Gruppe durch -O-, -O-CO-oder -CO-O- ersetzt sein kann und p 1, 2, 3, 4, 5 oder 6 ist, |
| $R^0$ | H oder $CH_3$, |

bedeutet,
mit der Maßgabe, daß $R^0$ von Y verschieden ist.

**3.** Fluorphenylpyrimidine nach Anspruch 2, wobei mindestens einer der Reste $R^1$ und $R^2$ eine Gruppe der Formel IIIa bedeutet,

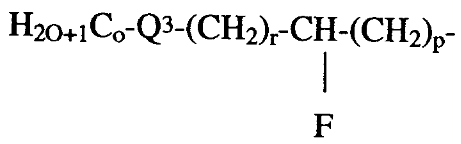

IIIa

worin o und $Q^3$ die angegebene Bedeutung besitzen, r 1 oder 2, p 0 oder 1 ist.

**4.** Nicht-chirale Fluorphenylpyrimidine nach Anspruch 1 der Formel I4,

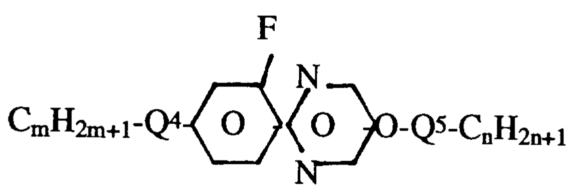

I4

worin

| | |
|---|---|
| m und n | jeweils unabhängig voneinander 1 bis 18, |
| $Q^4$ | -O- oder eine Einfachbindung |
| $Q^5$ | -CO-, |

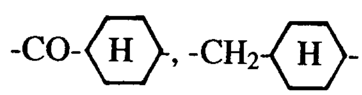

oder eine Einfachbindung bedeutet.

**5.** Verbindungen der Formel I4 nach Anspruch 4 ausgewählt aus den zweikernigen und dreikernigen Materialien der Formeln Ia bis If:

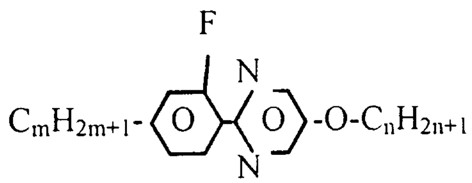

Ia

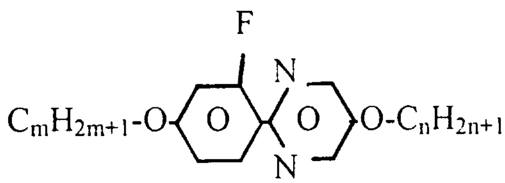

Ib

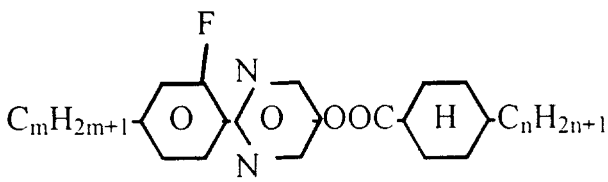

Ic

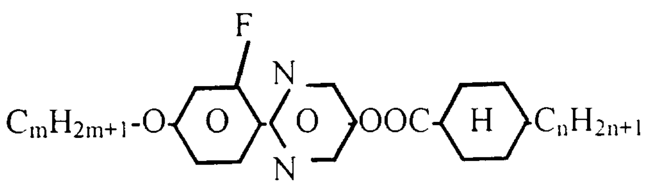

Id

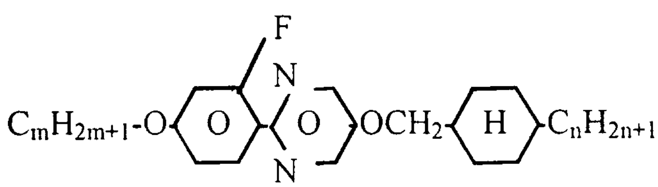

Ie

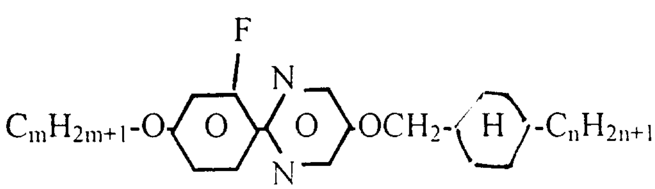

If

**6.** Chirale Verbindungen nach Anspruch 2 oder 3 ausgewählt aus den Verbindungen der Teilformeln Ik bis Ip mit 2 Ringen, wobei R* einen Rest der Formel III bedeutet:

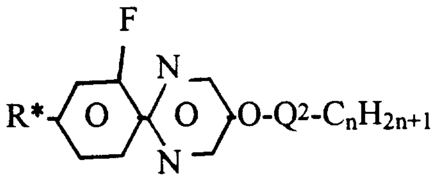

Ik

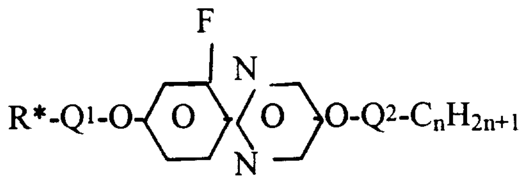

Il

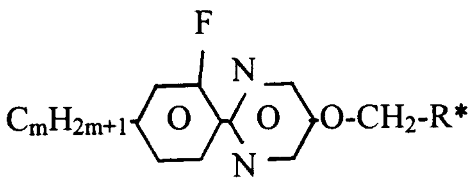

Im

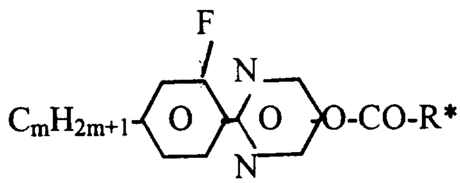

In

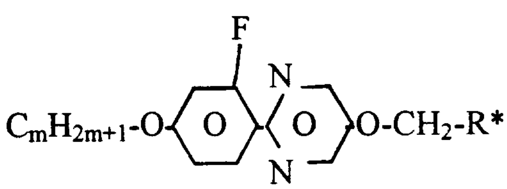

Io

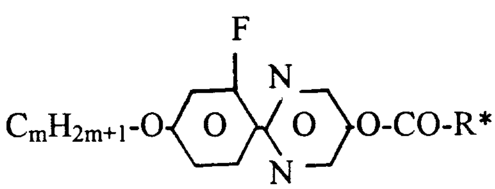

Ip

**7.** Chirale Verbindungen nach Anspruch 3 oder 6 wobei der chirale Rest IIIa ausgewählt ist aus den Resten der Formeln IIIa1 bis IIIa6:

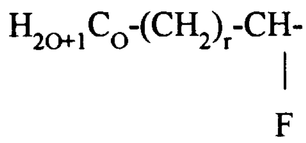
IIIa1

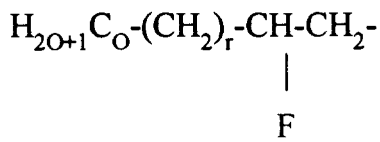
IIIa2

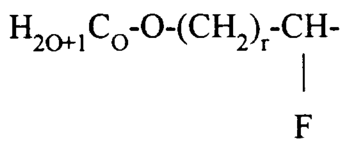
IIIa3

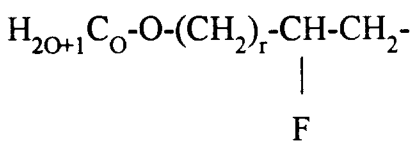
IIIa4

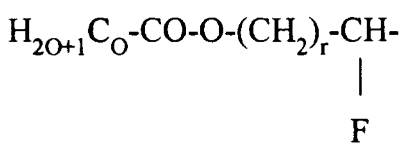
IIIa5

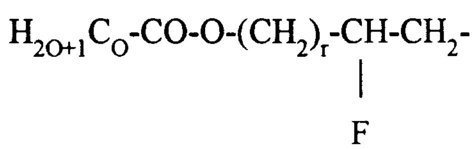
IIIa6

**8.** Flüssigkristallines Medium mit mindestens zwei flüssigkristallinen Komponenten, dadurch gekennzeichnet, daß mindestens eine Komponente eine Verbindung der Formel I ist.

**9.** Flüssigkristallanzeigeelement, dadurch gekennzeichnet, daß es ein flüssigkristallines Medium nach Anspruch 8 enthält.

**10.** Elektrooptisches Anzeigeelement, dadurch gekennzeichnet, daß es als Dielektrikum ein flüssigkristallines Medium nach Anspruch 8 enthält.

## Claims

1. Fluorophenylpyrimidines of the formula I:

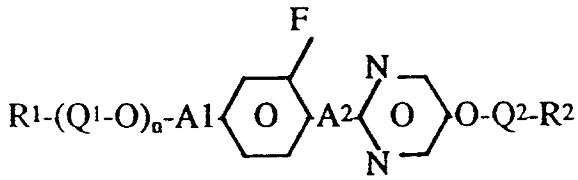

where

| | |
|---|---|
| $Q^1$ and $Q^2$ | are, in each case independently of each other, CO or $CH_2$, |
| n | is 0 or 1, |
| $A^1$ and $A^2$ | are, in each case independently of each other, 1,4-phenylene or a single bond, and |
| $R^1$ and $R^2$ | are, in each case independently of each other, an unsubstituted alkyl or alkenyl radical or an alkyl or alkenyl radical substituted by CN or by at least one halogen, which radical contains up to 18 carbon atoms and in which radical one or more $CH_2$ groups may be replaced by a radical selected from the group comprising -O-, -CO-O-, -O-CO- or -C≡C-, two oxygen atoms not being adjacent, |

and in the case where n = 1,
one of the radicals $R^1$ and $R^2$ may also be a group of the formula II:

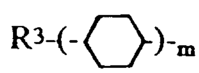

in which

| | |
|---|---|
| $R^3$ | is an unsubstituted alkyl, alkenyl or alkoxy group or an alkyl, alkenyl or alkoxy group substituted by CN or by at least one halogen, which group contains up to 18 carbon atoms, and |
| m | is 1 or 2. |

2. Fluorophenylpyrimidines of the formula I, in which at least one of the radicals $R^1$ and $R^2$ is a group of the formula III:

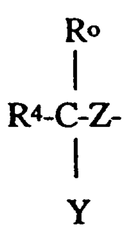

III

where

| | |
|---|---|
| $R^4$ | is a group of the formula $-(CH_2)_r-Q^3-C_oH_{2o+1}$, in which |
| r | is 0, 1 or 2, |
| o | is 1 to 7, and |
| $Q^3$ | is -O-, -O-CO- or a single bond, |
| Y | is CN, halogen or $CH_3$, |
| Z | is a single bond or $-(CH_2)_p-$ in which one $CH_2$ group may be replaced by -O-, -O-CO- or -CO-O- and p is 1, 2, 3, 4, 5 or 6, and |
| $R^{o}$ | is H or $CH_3$, |

with the proviso that R° is different from Y.

**3.** Fluorophenylpyrimidines according to Claim 2, in which at least one of the radicals $R^1$ and $R^2$ is a group of the formula IIIa:

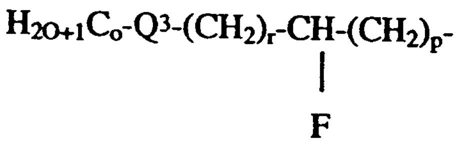

IIIa

in which o and $Q^3$ have the specified meaning, r is 1 or 2 and p is 0 or 1.

**4.** Non-chiral fluorophenylpyrimidines according to Claim 1 of the formula I4

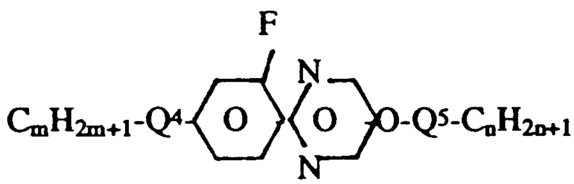

I4

in which

| | |
|---|---|
| m and n | are, in each case independently of one another, 1 to 18, |
| $Q^4$ | is -O- or a single bond, |
| $Q^5$ | is -CO-, |

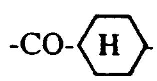 , 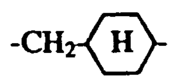

or a single bond.

**5.** Compounds of the formula I4 according to Claim 4 selected from the binuclear and trinuclear materials of the formulae Ia to If:

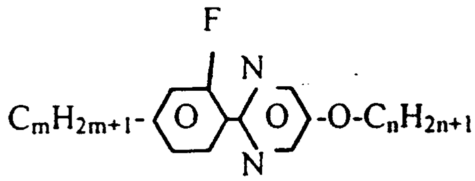

Ia

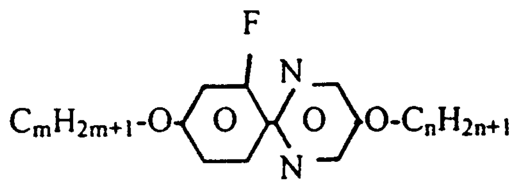

Ib

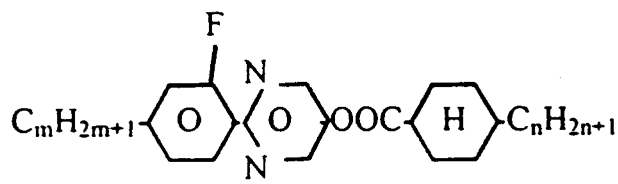

Ic

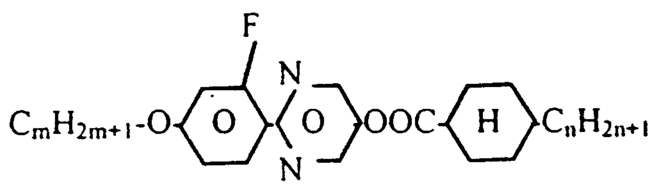

Id

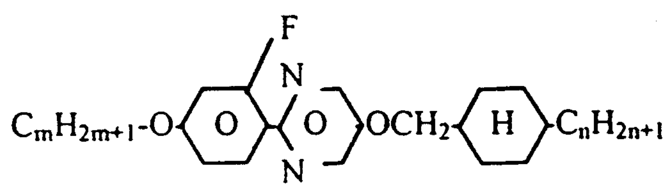

Ie

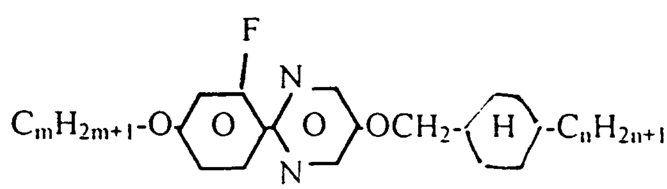

If

**6.** Chiral compounds according to Claim 2 or 3 selected from the compounds of the subformulae Ik to Ip and having 2 rings, where R* is a radical of the formula III:

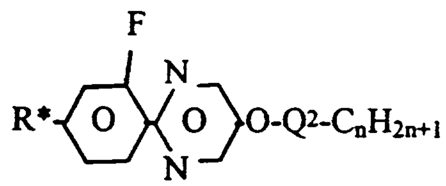

Ik

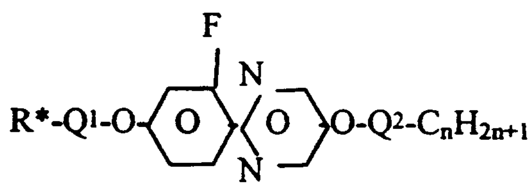

Il

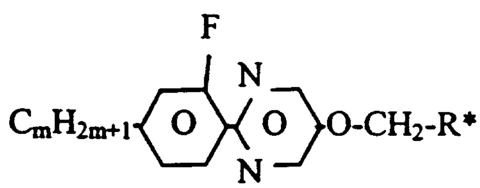

Im

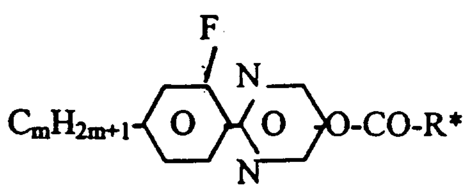

In

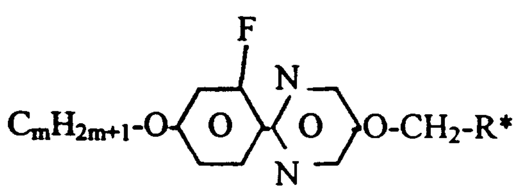

Io

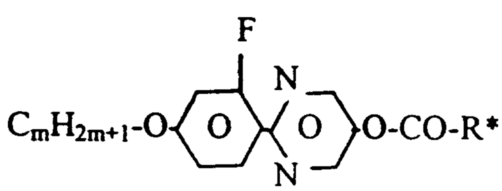

Ip

**7.** Chiral compounds according to Claim 3 or 6, where the chiral radical IIIa is selected from the radicals of the formulae IIIa1 to IIIa6:

$H_{2O+1}C_O$-$(CH_2)_r$-CH- IIIa1
|
F

$H_{2O+1}C_O$-$(CH_2)_r$-CH-$CH_2$- IIIa2
|
F

$H_{2O+1}C_O$-O-$(CH_2)_r$-CH- IIIa3
|
F

$H_{2O+1}C_O$-O-$(CH_2)_r$-CH-$CH_2$- IIIa4
|
F

$H_{2O+1}C_O$-CO-O-$(CH_2)_r$-CH- IIIa5
|
F

$H_{2O+1}C_O$-CO-O-$(CH_2)_r$-CH-$CH_2$- IIIa6
|
F

**8.** Liquid-crystalline medium containing at least two liquid-crystalline components, characterized in that at least one component is a compound of the formula I.

**9.** Liquid-crystal display component, characterized in that it contains a liquid-crystalline medium according to Claim 8.

**10.** Electro-optical display component, characterized in that it contains a liquid-crystalline medium according to Claim 8 as dielectric.

**Revendications**

**1.** Fluorophénylpyrimidines de formule I

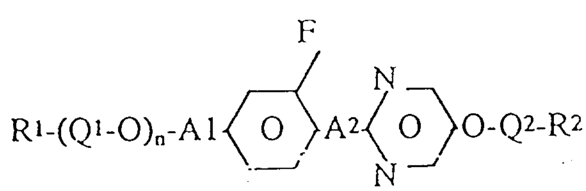

dans laquelle $Q^1$ et $Q^2$ représentent chacun, indépendamment l'un de l'autre, CO ou $CH_2$,
n est égal à 0 ou 1,
$A^1$ et $A^2$ représentent chacun, indépendamment l'un de l'autre, un groupe 1,4-phénylène ou un liaison simple et
$R^1$ et $R^2$ représentent chacun, indépendamment l'un de l'autre, un groupe alkyle ou alcényl non substitué ou substitué par un groupe CN ou par au moins un halogène et contenant jusqu'à 18 atomes de carbone, dans lequel un ou plusieurs groupes $CH_2$ peuvent être remplacés par un pont choisi parmi -O-, -CO-O-, -O-CO- ou -C = C, sous réserve qu'il ne peut y avoir deux atomes d'oxygène voisins,
et, lorsque n = 1,
l'un des symboles $R^1$ et $R^2$ peut également représenter un groupe de formule II

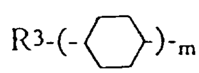

dans laquelle $R^3$ représente un groupe alkyle, alcényle ou alcoxy non substitué ou substitué par CN ou par au moins un halogène et contenant jusqu'à 18 atomes de carbone, et
m est égal à 1 ou 2.

**2.** Fluorophénylpyrimidines de formule I dans laquelle l'un au moins des symboles et $R^1$ représente un groupe de formule III

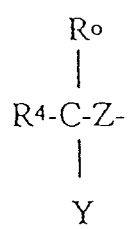
III

dans laquelle
$R^4$ représente un groupe de formule $-(CH_2)_r-Q_3-C_oH_{20+1}$,
dans lequel

- r est égal à O, 1 ou 2,
- o a une valeur de 1 à 7 et
- $Q^3$ représente -O-, -O-CO- ou une liaison simple,
- Y représente CH, un halogène ou $CH_3$,
- Z représente une liaison simple ou $-(CH_2)_p-$ dans lequel un groupe $CH_2$ peut être remplacé par -O-, -O-CO- ou -CO-O- et p est égal à 1, 2, 3, 4, 5 ou 6,
- $R^0$ représente H ou $CH_3$, sous réserve que $R^0$ et Y doivent être différents.

**3.** Fluorophénylpyrimidines selon revendication 2, pour lesquelles l'un au moins des symboles $R^1$ et $R^2$ représente un groupe de formule IIIa

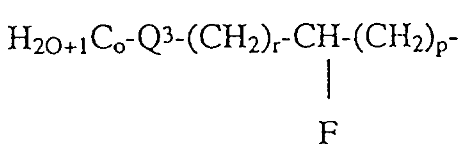
IIIa

dans laquelle o et $Q^3$ ont les significations indiquées ci-dessus, r est égal à 1 ou 2 et p à O ou 1.

**4.** Fluorophénylpyrimidines non chirales selon revendication 1, de formule I4

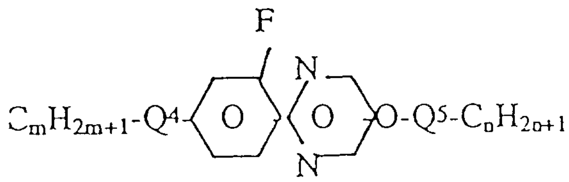

I4

dans laquelle m et n sont des nombres allant chacun, indépendamment l'un de l'autre, de 1 à 18,

$Q^4$ représente -0- ou une liaison simple,

$Q^5$ représente -CO-,

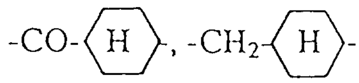

ou une liaison simple.

**5.** Composés de formule I4 selon revendication 4, choisis parmi les substances bicycliques et tricycliques de formules Ia à If

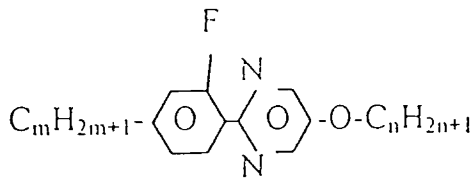

Ia

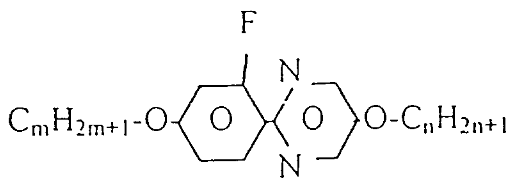

Ib

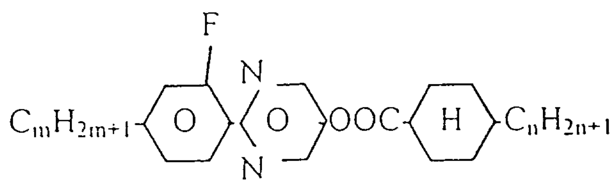

Ic

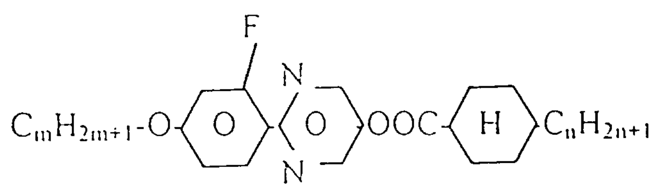

Id

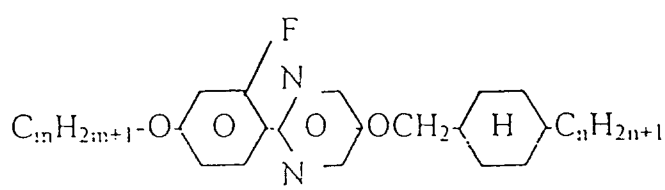

Ie

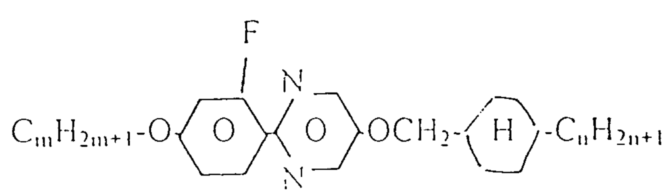

If

**6.** Composés chiraux selon revendication 2 ou 3, choisis parmi les composés répondant aux formules partielles Ik à Ip, à deux cycles et dans lesquelles R* représente un groupe de formule III

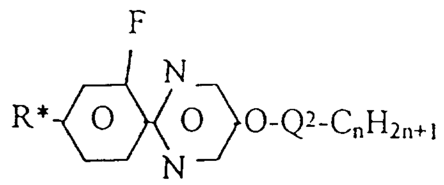

Ik

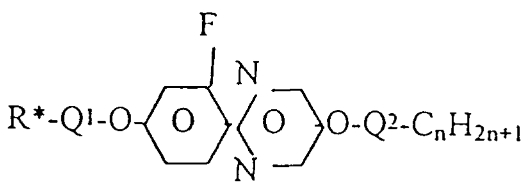

Il

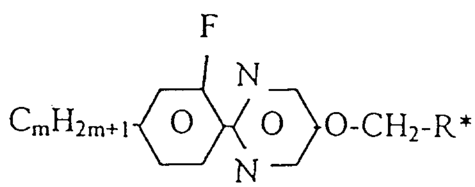

Im

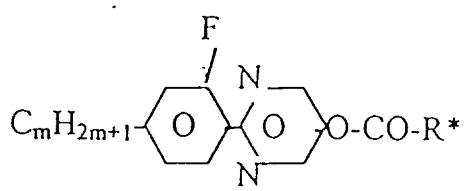

In

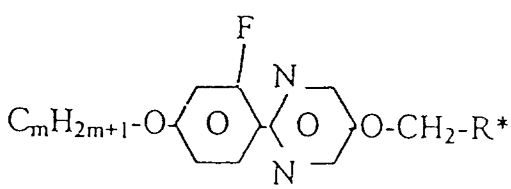

Io

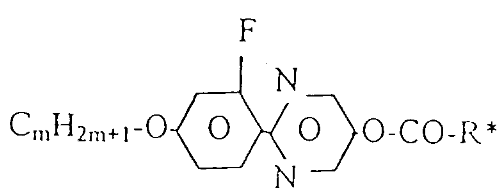

Ip

7. Composés chiraux selon revendication 3 ou 6, pour lesquels le radical chiral IIIa est choisi parmi les radicaux de formule IIIa 1 à IIIa 6.

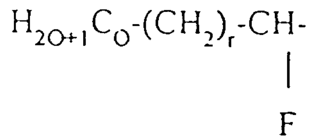
IIIa1

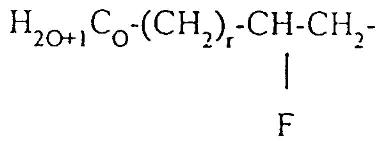
IIIa2

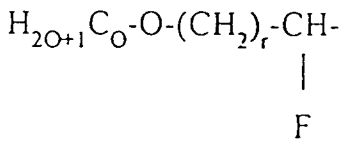
IIIa3

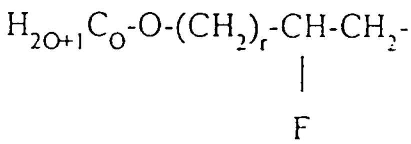
IIIa4

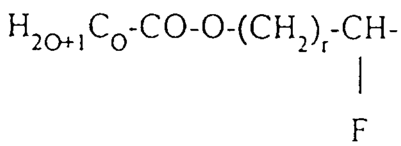
IIIa5

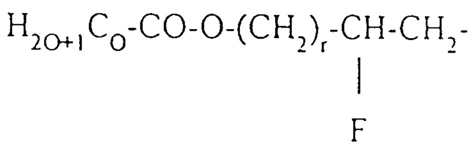
IIIa6

**8.** Milieu à cristaux liquides à au moins deux composants à cristaux liquides, caractérisé en ce que l'un au moins des composants est un composé de formule I.

**9.** Elément d'affichage à cristaux liquides, caractérisé en ce qu'il contient un milieu à cristaux liquides selon revendication 8.

**10.** Elément d'affichage électrooptique, caractérisé en ce qu'il contient en tant que diélectrique un milieu à cristaux liquides selon revendication 8.